# EUROPEAN PATENT APPLICATION

(11) **EP 3 415 631 A1**
(43) Date of publication of application: **19.12.2018**
(21) Application number: 17175730.5
(22) Date of filing: 13.06.2017
(51) Int. Cl.: C12P 19/02, C12N 9/04

(54) **METHODS OF PRODUCING 5-KETOFRUCTOSE**

(71) Applicant: RWTH Aachen, 52062 Aachen (DE); Forschungszentrum Jülich GmbH, 52425 Jülich (DE); Rheinische Friedrich-Wilhelms-Universität Bonn, 53113 Bonn (DE)
(72) Inventor: Herweg, Elena, 52066 Aachen (DE); Büchs, Prof. Dr. Jochen, 52064 Aachen (DE); Bott, Prof. Dr. Michael, 52428 Jülich (DE); Kiefler, Dr. Ines, 41179 Mönchengladbach (DE); Deppenmeier, Prof. Dr. Uwe, 53797 Lohmar (DE); Kosciow, Konrad, 53111 Bonn (DE); Siemen, Anna, 50670 Köln (DE)
(74) Representative: Lasar, Andrea Gisela

(57) **Abstract**

The present invention relates to a method of producing 5-ketofructose using an acetic acid bacterium which is genetically modified to express a fructose dehydrogenase as well as to microorganisms which can be used in said method.

## Description

### FIELD OF THE INVENTION

The present invention relates to a method of producing 5-ketofructose using an acetic acid bacterium which is genetically modified to express a fructose dehydrogenase as well as to microorganisms which can be used in said method.

### BACKGROUND OF THE INVENTION

Low-calorie sweeteners provide consumers with both psychological and physiological benefits. For example, low-calorie sweeteners are believed to be effective for weight maintenance, weight reduction, management of diabetes, reduction of dental caries, and reduction in the risks associated with obesity. However, they are also used as part of an overall healthy lifestyle. Low-calorie sweeteners which are present in currently available food products include aspartame, acesulfame K, sugar alcohols, D-tagatose, steviol glycosides and maple syrup. However, at present there is no natural, low-calorie sweetener which has good sensoric properties and which cannot be metabolized by intestinal bacteria. For example, the sweetener composition Stevia obtained from the plant *Stevia rebaudiana* has a licorice-like taste and is therefore not suitable for all applications.

In contrast, 5-ketofructose cannot be metabolized by intestinal bacteria, has a natural sweetness and a high sweetening intensity in the same range as fructose.
DE 10 2007 026 713 discloses that 5-ketofructose produced *in vitro* using a purified 5-D-fructose dehydrogenase can be used as a sweetener. The 5-ketofructose may also be used as a starting material for the synthesis of pyrrolidine aza sugars which can be used pharmaceutically as glycohydrolase inhibitors (Baxter and Reitz (1994) J. Org. Chem. 59: 3175-3185).

Methods for producing 5-ketofructose have been described in the art. For example, L-sorbose can be converted to 5-ketofructose *in vitro* using the purified enzyme pyranose-2-oxidase, yielding up to 180 g/l 5-ketofructose (Schneider et al. (2012) Biotechnol. Bioeng. 109: 2941-2945). For regenerating the co-substrate oxygen catalases have to be used. Since the enzymes required in this process need to be produced and purified, this method is expensive and time-consuming.

US 3 206 375 A describes the conversion of fructose to 5-ketofructose in different wild-type strains of the genus Acetobacter, yielding 10 to 16 g purified 5-ketofructose from 100 g/l fructose. However, this method is inefficient, since only a small part of the fructose is converted slowly to 5-ketofructose. Further, the wild-type strains produce overflow metabolites such as acetate, further reducing the product yield.

Hence, there is still a need for an efficient method of producing 5-ketofructose.

### SUMMARY OF THE INVENTION

The present inventors have surprisingly found that 5-ketofructose can be produced efficiently in acetic acid bacteria which are genetically modified to express a recombinant fructose dehydrogenase, using fructose, glucose, saccharose or starch as the starting material for the production of 5-ketofructose.
Accordingly, the present invention relates to a method for producing 5-ketofructose in an acetic acid bacterium, comprising the steps of:
a) contacting a first acetic acid bacterium which is genetically modified to express a fructose dehydrogenase with a solution comprising a carbohydrate; and
b) isolating the 5-ketofructose.

Preferably, the acetic acid bacterium is *Gluconobacter oxydans.*

In one embodiment step (a) comprises culturing the acetic acid bacterium in a culture medium comprising said carbohydrate.

In one embodiment, the expression vector further comprises a promoter region from Gluconobacter.

In one embodiment, the carbohydrate is fructose.

In one embodiment, the acetic acid bacterium is cultured in fed-batch mode in a culture medium comprising fructose.

In one embodiment, the culture medium comprises 30 to 275 g/l fructose.

In one embodiment, the carbohydrate is glucose and the glucose is converted to fructose by the action of a glucose isomerase, and wherein the acetic acid bacterium lacks expression of a membrane-bound glucose dehydrogenase.

In one embodiment, the carbohydrate is sucrose and the sucrose is converted to fructose by the action of a glucose isomerase and an invertase and wherein the acetic acid bacterium lacks expression of a membrane-bound glucose dehydrogenase.

In one embodiment, the carbohydrate is starch and the starch is converted to fructose by the action of an amylase, a pullulanase and a glucose isomerase and wherein the acetic acid bacterium lacks expression of a membrane-bound glucose dehydrogenase.

The present invention further relates to a method for producing a sweetener composition, comprising performing the method for producing 5-ketofructose as described herein and preparing a sweetener composition comprising 5-ketofructose.

The present invention further relates to the use of an acetic acid bacterium which is genetically modified to express a fructose dehydrogenase for producing 5-ketofructose.

The present invention further relates to a *Gluconobacter oxydans* cell containing:
(a) an expression vector comprising a nucleic acid sequence which encodes a fructose dehydrogenase and a glucose isomerase or
(b) a first expression vector comprising a nucleic acid sequence which encodes a fructose dehydrogenase and a second expression vector comprising a nucleic acid sequence which encodes a glucose isomerase.

In one embodiment, the expression vector of (a) further comprises a nucleic acid sequence which encodes an invertase or (b) further containing a third expression vector comprising a nucleic acid sequence which encodes an invertase.

In one embodiment, the expression vector of (a) further comprises a nucleic acid sequence which encodes an amylase and a pullulanase or (b) further containing a third expression vector comprising a nucleic acid sequence which encodes an amylase and a fourth expression vector comprising a nucleic acid sequence which encodes a pullulanase.

### BRIEF DESCRIPTION OF THE DRAWINGS

**Figure 1****:** Conversion of fructose to 5-ketofructose in the *G. oxydans* wild-type strain and in a recombinant *G. oxydans* strain expressing fructose dehydrogenase
**Figure 2****:** Production of 5-ketofructose from fructose in a fed-batch process
**Figure 3****:** Production of 5-ketofructose from sucrose in a mixed culture of bacteria

### DETAILED DESCRIPTION OF THE INVENTION

Although the present invention will be described with respect to particular embodiments, this description is not to be construed in a limiting sense.

Before describing in detail exemplary embodiments of the present invention, definitions important for understanding the present invention are given. As used in this specification and in the appended claims, the singular forms of "a" and "an" also include the respective plurals unless the context clearly dictates otherwise. In particular, the term "acetic acid bacterium" typically includes a composition comprising several single acetic acid bacterial organisms. In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %. It is to be understood that the term "comprising" is not limiting. For the purposes of the present invention the term "consisting of" is considered to be a preferred embodiment of the term "comprising". If hereinafter a group is defined to comprise at least a certain number of embodiments, this is meant to also encompass a group which preferably consists of these embodiments only. Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein. In case the terms "first", "second", "third" or "(a)", "(b)", "(c)", "(d)", "i", "ii" etc. relate to steps of a method or use or assay there is no time or time interval coherence between the steps, i.e. the steps may be carried out simultaneously or there may be time intervals of seconds, minutes, hours, days, weeks, months or even years between such steps, unless otherwise indicated in the application as set forth herein above or below.

It is to be understood that this invention is not limited to the particular methodology, protocols, reagents etc. described herein as these may vary. It is also to be understood that the terminology used herein is for the purpose of describing particular embodiments only, and is not intended to limit the scope of the present invention that will be limited only by the appended claims. Unless defined otherwise, all technical and scientific terms used herein have the same meanings as commonly understood by one of ordinary skill in the art.

As discussed above, the present invention is based on the finding that 5-ketofructose can be efficiently produced in an acetic acid bacterium genetically modified to express a fructose dehydrogenase.

5-ketofructose which can be produced from fructose by a dehydrogenation reaction has the following structure: 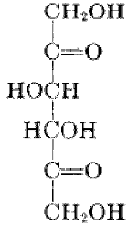 The 5-ketofructose produced by the method of the present invention can be used as a sweetener or for the synthesis of pyrrolidine aza sugars.

In the methods of the present invention the 5-ketofructose is produced in an acetic acid bacterium. Acetic acid bacteria are strictly aerobic gram-negative bacteria which oxidize sugars or ethanol and produce acetic acid during fermentation. Within the present invention, the term "acetic acid bacterium" is intended to comprise a bacterium from a genus selected from the group consisting of *Acetobacter*, *Acidomonas*, *Ameyamaea*, *Asaia*, *Gluconacetobacter*, *Gluconobacter*, *Granulibacter*, *Kozakia*, *Neoasaia*, *Neokomagataea*, *Saccharibacter*, *Swaminathania* and *Tanticharoenia.*

Preferably, the acetic acid bacterium is from the genus Gluconobacter. The genus Gluconobacter includes, but is not limited to, the species *Gluconobacter albidus, Gluconobacter asaii, Gluconobacter cerevisiae, Gluconobacter cerinus, Gluconobacter frateurii, Gluconobacter japonicus, Gluconobacter kanchanaburiensis, Gluconobacter kondonii, Gluconobacter nephelii, Gluconobacter oxydans, Gluconobacter sphaericus, Gluconobacter thailandicus, Gluconobacter uchimurae* and *Gluconobacter wancherniae.*

More preferably, the acetic acid bacterium is *Gluconobacter oxydans.* Different strains of *Gluconobacter oxydans* are available to the skilled person, including *Gluconobacter oxydans* DSM 4025, *Gluconobacter oxydans* DSM 3503, *Gluconobacter oxydans* NBIMCC 1043, *Gluconobacter oxydans* ATCC 621, *Gluconobacter oxydans* NBIMCC 902, *Gluconobacter oxydans* CCM 3607 and *Gluconobacter oxydans* CCM 1783. Most preferably, *Gluconobacter oxydans* ATCC 621 is used in the methods of the present invention.

The acetic acid bacterium used in the methods of the present invention is genetically modified to express a fructose dehydrogenase. The term "genetically modified acetic acid bacterium" or "acetic acid bacterium which is genetically modified" as used herein means that an acetic acid bacterium is altered by any suitable genetic means and methods known to the skilled person in order to express a fructose dehydrogenase or to increase the expression of fructose dehydrogenase in the acetic acid bacterium. Methods for genetically modifying acetic acid bacteria are known to the person skilled in the art and are described in the literature (see, e.g., Kallnik et al. (2010) J. Biotechnol. 150: 460-465; Kostner et al. (2013) Appl. Microbiol. Biotechnol. 97: 8341-8349; Kosciow et al. (2014) J. Biotechnol. 189: 27-35; Kiefler et al. (2017) Appl. Microbiol. Biotechnol. in press). They comprise commonly used methods for introducing genetic elements or material into the acetic acid bacterium so as to be contained in the acetic acid bacterium, integrated into the chromosome or extrachromosomally, or the removal or destruction, or modification, of genetic elements or sequences present in the genome of a wild-type acetic acid bacterium.

The genetically modified acetic acid bacterium can be distinguished from an acetic acid bacterium which is not genetically modified in that it expresses a fructose dehydrogenase which is not naturally expressed in the acetic acid bacterium which is genetically modified. Alternatively, the expression of a fructose dehydrogenase which is naturally expressed in the acetic acid bacterium which is genetically modified is increased in the genetically modified acetic acid bacterium compared to the acetic acid bacterium which is not genetically modified. Further, the genetically modified acetic acid bacterium can be distinguished from an acetic acid bacterium which is not genetically modified by the presence of the genetic element which leads to an increased expression of fructose dehydrogenase. If the genetic element leading to an increased expression of fructose dehydrogenase is an expression vector comprising a nucleic acid sequence encoding the fructose dehydrogenase, the genetically modified acetic acid bacterium can be distinguished from an acetic acid bacterium which is not genetically modified by the presence of this expression vector within the cell. For example, it can be determined that the nucleic acid sequence encoding the fructose dehydrogenase is operably linked to regulatory sequences such as a promoter to which it is not naturally linked in the organism from which the nucleic acid sequence encoding the fructose dehydrogenase is derived.

Preferably, the acetic acid bacterium is genetically modified with an expression vector encoding the fructose dehydrogenase.

As used herein, the term "vector" refers to a nucleic acid molecule capable of transporting another nucleic acid to which it has been linked and may be used herein interchangeably with the term "recombinant nucleic acid molecule". One type of vector is a "plasmid", which refers to a circular double stranded DNA loop into which additional DNA segments can be ligated. In the present specification, "plasmid" and "vector" can be used interchangeably as the plasmid is the most commonly used form of vector. The term "expression vector" means a vector capable of directing expression of a particular nucleotide sequence in an appropriate host cell. An expression vector comprises a regulatory nucleic acid element operably linked to a nucleic acid of interest, which is - optionally - operably linked to a termination signal and/or other regulatory element.

The term "genetic element" as used herein means any molecular unit which is able to transport genetic information. It accordingly relates to a gene, preferably to a native gene, a chimeric gene, a foreign gene, a transgene or a codon-optimized gene. The term "gene" refers to a nucleic acid molecule or fragment that expresses a specific protein, preferably it refers to nucleic acid molecules including regulatory sequences preceding (5' non-coding sequences) and following (3' non-coding sequences) the coding sequence. The term "native gene" refers to a gene as found in nature, e.g. in a wild-type strain of an acetic acid bacterium, with its own regulatory sequences. The term "chimeric gene" refers to any gene that is not a native gene, comprising regulatory and coding sequences that are not found together in nature. Accordingly, a chimeric gene may comprise regulatory sequences and coding sequences that are derived from different sources, or regulatory sequences and coding sequences derived from the same source, but arranged in a manner different than that found in nature. According to the present invention a "foreign gene" refers to a gene not normally found in the acetic acid bacterium, but that is introduced into the acetic acid bacterium by gene transfer. Foreign genes can comprise native genes inserted into a non-native organism, or chimeric genes. The term "transgene" refers to a gene that has been introduced into the genome by a transformation procedure.

The term "coding sequence" refers to a DNA sequence which codes for a specific amino acid sequence. The term "regulatory sequence" refers to a nucleotide sequence located upstream (5' non-coding sequences), within, or downstream (3' non-coding sequences) of a coding sequence, and which influences the transcription, RNA processing or stability, or translation of the associated coding sequence. Regulatory sequences may include promoters, enhancers, translation leader sequences, introns, polyadenylation recognition sequences, RNA processing sites, effector binding sites and stem-loop structures.

The term "promoter" refers to a DNA sequence capable of starting and controlling the expression of a coding sequence or functional RNA. Typically, a coding sequence is located 3' to a promoter sequence. Promoters may be derived in their entirety from a native gene, or be composed of different elements derived from different promoters found in nature, or even comprise synthetic DNA segments. Typically, since the exact boundaries of regulatory sequences have not been completely defined, DNA fragments of different lengths may have identical promoter activity. It is understood by a person skilled in the art that different promoters may direct the expression of a gene at different stages of development, or in response to different environmental or physiological conditions. Promoters that cause a gene to be expressed in most cell types at most times are commonly referred to as constitutive promoters. On the other hand, promoters that cause a gene to be expressed in specific contexts only, e.g. based on the presence of specific factors, growth stages, temperatures, pH or the presence of specific metabolites etc. are understood as regulable promoters.

The term "3' non-coding sequences" refers to DNA sequences located downstream of a coding sequence. The term "RNA transcript" refers to the product resulting from RNA polymerase catalyzed transcription of a DNA sequence. The term "mRNA" refers to messenger RNA, i.e. RNA that is without introns and that can be translated into protein by the cell.

The term "operably linked" refers to the association of nucleic acid sequences on a single nucleic acid fragment so that the function of one is affected by the other. In the context of a promoter the term means that the coding sequence is under the transcriptional control of the promoter which regulates the expression of the coding sequence.

Regulatory elements for driving expression of genes in acetic acid bacteria are known to the person skilled in the art and include constitutive promoters of genes encoding ribosomal proteins, preferably the promoter is the p264 or the p452 promoter of Gluconobacter (see Kallnik et al. (2010) J. Biotechnol. 150: 460-465). More preferably, the promoter is the p264 promoter.

Even more preferably, the promoter has a nucleic acid sequence selected from the group consisting of:
a) the nucleic acid sequence according to SEQ ID No. 7;
b) a functional part of the nucleic acid sequence according to SEQ ID No. 7; and
c) a nucleic acid sequence hybridizing under stringent conditions with a complementary sequence of the nucleic acid sequence according to SEQ ID No. 7.

The functional part of the nucleic acid sequence according to SEQ ID No. 7 is able to drive expression of a nucleic acid sequence which is operably linked to it substantially to the same extent as the full-length sequence according to SEQ ID No. 7, i.e. the expression level of the nucleic acid sequence which is linked to the functional part is at least 75%, preferably at least 80% or 85%, more preferably at least 90% or 92% and most preferably at least 95% of the expression level of said nucleic acid sequence when linked to the full-length promoter sequence according to SEQ ID No. 7.

The term "hybridizing under stringent conditions" denotes in the context of the present invention that the hybridization is implemented *in vitro* under conditions which are stringent enough to ensure a specific hybridization. Stringent *in vitro* hybridization conditions are known to those skilled in the art and may be taken from the literature (e.g. Sambrook and Russell (2001) Molecular Cloning: A Laboratory Manual, 3rd edition, Cold Spring Harbour Laboratory Press, Cold Spring Harbour, NY). The term "specific hybridization" refers to the circumstance that a molecule, under stringent conditions, preferably binds to a certain nucleic acid sequence, i.e. the target sequence, if the same is part of a complex mixture of, e.g. DNA or RNA molecules, but does not, or at least very rarely, bind to other sequences.

Stringent conditions depend on the circumstances. Longer sequences hybridize specifically at higher temperatures. In general, stringent conditions are chosen such that the hybridization temperature is about 5°C below the melting point (Tₘ) of the specific sequence at a defined ionic strength and at a defined pH value. Tₘ is the temperature (at a defined pH value, a defined ionic strength and a defined nucleic acid concentration), at which 50% of the molecules complementary to the target sequence hybridize to the target sequence in the state of equilibrium. Typically, stringent conditions are conditions, where the salt concentration has a sodium ion concentration (or concentration of a different salt) of at least about 0.01 to 1.0 M at a pH value between 7.0 and 8.3, and the temperature is at least 30°C for small molecules (i.e. 10 to 50 nucleotides, for example). In addition, stringent conditions may include the addition of substances, such as, e. g., formamide, which destabilise the hybrids. At hybridization under stringent conditions, as used herein, normally nucleotide sequences which are at least 60% homologous to each other hybridize to each other. Preferably, said stringent conditions are chosen such that sequences which are about 65%, preferably at least about 70%, and especially preferably at least about 75% or higher homologous to each other, normally remain hybridized to each other. A preferred but non-limiting example of stringent hybridization conditions is hybridizations in 6 x sodium chloride/sodium citrate (SSC) at about 45°C, followed by one or more washing steps in 0.2 x SSC, 0.1% SDS at 50 to 65°C. The temperature depends on the type of the nucleic acid and is between 42°C and 58°C in an aqueous buffer having a concentration of 0.1 to 5 x SSC (pH value 7.2).

If an organic solvent, e.g. 50% formamide, is present in the above-mentioned buffer, the temperature is about 42°C under standard conditions. Preferably, the hybridisation conditions for DNA:DNA hybrids are, for example, 0.1 x SSC and 20°C to 45°C, preferably 30°C to 45°C. Preferably, the hybridisation conditions for DNA:RNA hybrids are, for example, 0.1 x SSC and 30°C to 55°C, preferably between 45°C and 55°C. The above-mentioned hybridization temperatures are determined, for example, for a nucleic acid which is 100 base pairs long and has a G/C content of 50% in the absence of formamide. Those skilled in the art know how to determine the required hybridization conditions using text books such as those mentioned above or the following textbooks: Current Protocols in Molecular Biology, John Wiley & Sons, N.Y. (1989), Hames and Higgins (publ.) 1985, Nucleic Acids Hybridization: A Practical Approach, IRL Press at Oxford University Press, Oxford; Brown (publ.) 1991, Essential Molecular Biology: A Practical Approach, IRL Press at Oxford University Press, Oxford.

Typical hybridization and washing buffers for example have the following composition:

| | |
|---|---|
| *Pre-hybridization solution:* | 0.5 % SDS |
| | 5x SSC |
| | 50 mM NaPO₄, pH 6.8 |
| | 0.1 % sodium pyrophosphate |
| | 5x Denhardt's solution |
| | 100 µg/mL salmon sperm DNA |
| | |
| *Hybridization solution:* | pre-hybridization solution |
| | 1x10⁶ cpm/mL probe (5 - 10 min 95 °C) |
| | |
| *20x SSC:* | 3 M NaCl |
| | 0.3 M sodium citrate |
| | ad pH 7 with HCl |
| | |
| *50x Denhardt's reagent:* | 5 g Ficoll |
| | 5 g polyvinylpyrrolidone |
| | 5 g bovine serum albumin |
| | ad 500 mL aqua destillata |

A typical procedure for hybridization is as follows:

| | | | |
|---|---|---|---|
| *Optional:* | | wash blot 30 min in 1x SSC/ 0.1 % SDS at 65 °C | |
| | | | |
| *Pre-hybridization:* | | at least 2 h at 50 - 55 °C | |
| | | | |
| *Hybridization:* | | over night at 55 - 60 °C | |
| *Washing:* | 05 min | 2x SSC/ 0.1 % SDS | hybridization temp. |
| | 30 min | 2x SSC/ 0.1 % SDS | hybridization temp. |
| | 30 min | 1x SSC/ 0.1 % SDS | hybridization temp. |
| | 45 min | 0.2x SSC/ 0.1 % SDS | 65 °C |
| | 5 min | 0.1x SSC | room temperature |

Those skilled in the art know that the given solutions and the presented protocol may be modified or have to be modified, depending on the application.

In the present invention, an acetic acid bacterium is genetically modified to express a fructose dehydrogenase. The fructose dehydrogenase catalyzes the oxidation of fructose to 5-ketofructose. The fructose dehydrogenase may be from *Gluconobacter japonicus, Gluconobacter frateurii, Gluconobacter albidus, Gluconobacter cerinus, Neokomagataea thailandica, Frateuria aurantia* or *Tatumella sp.* It is a heterotrimeric membrane-bound enzyme which is composed of three subunits which are encoded by separate nucleic acid sequences.

Preferably, the fructose dehydrogenase used in the methods of the present invention is from *Gluconobacter japonicus.* It is composed of the three subunits FdhS, FdhC and FdhL which are encoded by separate nucleic acid sequences.

The subunit FdhS is preferably encoded by a nucleic acid sequence selected from the group consisting of:
a) a nucleic acid sequence comprising the sequence according to SEQ ID No. 1 or a fragment thereof;
b) a nucleic acid sequence encoding a protein comprising the amino acid sequence according to SEQ ID No. 2;
c) a nucleic acid sequence comprising a sequence which is at least 70 % identical to the sequence according to SEQ ID No. 1 or a fragment thereof;
d) a nucleic acid sequence hybridizing under stringent conditions with a complementary sequence of a nucleic acid sequence according to SEQ ID No. 1 or a fragment thereof; and
e) a nucleic acid sequence encoding the same fructose dehydrogenase as any of the nucleic acid sequences of (a) to (d) above, but differing from the nucleic acid sequences of (a) to (d) above due to the degeneracy of the genetic code.

The subunit FdhC is preferably encoded by a nucleic acid sequence selected from the group consisting of:
a) a nucleic acid sequence comprising the sequence according to SEQ ID No. 3 or a fragment thereof;
b) a nucleic acid sequence encoding a protein comprising the amino acid sequence according to SEQ ID No. 4;
c) a nucleic acid sequence comprising a sequence which is at least 70 % identical to the sequence according to SEQ ID No. 3 or a fragment thereof;
d) a nucleic acid sequence hybridizing under stringent conditions with a complementary sequence of a nucleic acid sequence according to SEQ ID No. 3 or a fragment thereof; and
e) a nucleic acid sequence encoding the same fructose dehydrogenase as any of the nucleic acid sequences of (a) to (d) above, but differing from the nucleic acid sequences of (a) to (d) above due to the degeneracy of the genetic code.

The subunit FdhL is preferably encoded by a nucleic acid sequence selected from the group consisting of:
a) a nucleic acid sequence comprising the sequence according to SEQ ID No. 5 or a fragment thereof;
b) a nucleic acid sequence encoding a protein comprising the amino acid sequence according to SEQ ID No. 6;
c) a nucleic acid sequence comprising a sequence which is at least 70 % identical to the sequence according to SEQ ID No. 5 or a fragment thereof;
d) a nucleic acid sequence hybridizing under stringent conditions with a complementary sequence of a nucleic acid sequence according to SEQ ID No. 5 or a fragment thereof; and
e) a nucleic acid sequence encoding the same fructose dehydrogenase as any of the nucleic acid sequences of (a) to (d) above, but differing from the nucleic acid sequences of (a) to (d) above due to the degeneracy of the genetic code.

A "fragment" of the nucleic acid sequence according to any one of SEQ ID Nos. 1, 3 or 5 is understood to refer to a smaller part of this nucleic acid sequence which consists of a contiguous nucleotide sequence found in SEQ ID No. 1, 3 or 5 and which encodes a protein having the activity of a fructose dehydrogenase when expressed with the other two subunits of fructose dehydrogenase as specified above.

In case the fragment is described to be a fragment of a sequence with a certain degree of sequence identity to a particular sequence, the fragment shall be a fragment of the sequence which has a certain degree of sequence identity to the particular sequence. Thus, for instance, in expressions like "a nucleic acid sequence comprising a sequence which is at least 70% identical to the sequence according to SEQ ID No. 1, 3 or 5 or a fragment thereof" the "fragment" in the second alternative refers to a fragment of the sequence which sequence is at least 70% identical to the sequence according to SEQ ID No. 1, 3 or 5.

The fragment of SEQ ID No. 1 has a length of at least 300 nucleotides, preferably of at least 320, 340 or 360 nucleotides, more preferably of at least 380, 400 and 420 nucleotides and most preferably of at least 440, 460, 480, 500, 520 or 540 nucleotides. The fragment of SEQ ID No. 2 has a length of at least 1000 nucleotides, preferably of at least 1050 or 1100 nucleotides, more preferably of at least 1150, 1200 or 1250 nucleotides and most preferably of at least 1300, 1350 or 1400 nucleotides. The fragment of SEQ ID No. 3 has a length of at least 1000, 1050, 1100 or 1150 nucleotides, preferably of at least 1200, 1250 or 1300 nucleotides, more preferably of at least 1350, 1400 or 1450 nucleotides and most preferably of at least 1500, 1550 or 1600 nucleotides.

The present invention further relates to the use of nucleic acid sequences which are at least 70%, 75% or 80 % identical, preferably at least 81, 82, 83, 84, 85 or 86% identical, more preferably at least 87, 88, 89 or 90% identical, even more preferably at least 91, 92, 93, 94 or 95% identical and most preferably at least 96, 97, 98, 99 or 100% identical to the complete sequence according to any of SEQ ID Nos. 1, 3 or 5 or a fragment of any of these sequences and which encode a protein having the activity of a fructose dehydrogenase when expressed with the other two subunits of fructose dehydrogenase as specified above.

Within the meaning of the present invention, "sequence identity" denotes the degree of conformity with regard to the 5' - 3' sequence within a nucleic acid molecule in comparison to another nucleic acid molecule. Preferably, the "percentage of sequence identity" is calculated by comparing two optimally aligned sequences over a particular region, determining the number of positions at which the identical base or amino acid is present in both sequences in order to yield the number of matched positions, dividing the number of those matched positions by the total number of positions in the segment being compared and multiplying the result by 100. The sequence identity may be determined using a series of programs, which are based on various algorithms, such as BLASTN, ScanProsite, the laser gene software, etc. As an alternative, the BLAST program package of the National Center for Biotechnology Information (http://www.ncbi.nlm.nih.gov/) may be used applying the default parameters. Here, in addition, the program Sequencher (Gene Codes Corp., Ann Arbor, MI, USA) using the "dirtydata"-algorithm for sequence comparisons was employed.

The sequence identity refers to the degree of the sequence identity over a length of at least 300 nucleotides, preferably of 320, 340, 360, 380, 400 or 420 nucleotides and more preferably of 440, 460, 480, 500, 520 or 540 nucleotides and most preferably the whole length of SEQ ID No. 1. The sequence identity refers to the degree of the sequence identity over a length of 1000 nucleotides, preferably of 1050, 1100, 1150 or 1200 nucleotides, more preferably of 1250, 1300, 1350 or 1400 nucleotides and most preferably over the whole length of SEQ ID No. 3. The sequence identity refers to the degree of the sequence identity over a length of 1000, 1050, 1100, 1150, 1200 or 1250 nucleotides, preferably of 1300, 1350, 1400 or 1450 nucleotides, more preferably of 1500, 1550 or 1600 nucleotides and most preferably the whole length of SEQ ID No. 5.

The nucleic acid sequence hybridizing under stringent conditions with a complementary sequence of a nucleic acid sequence according to any of SEQ ID Nos. 1, 3 or 5 or a fragment thereof encodes a protein having the activity of a fructose dehydrogenase when expressed with the other two subunits of fructose dehydrogenase as specified above.

The variants of the sequences according to SEQ ID Nos. 1, 3 and 5 have essentially the same activity as the fructose dehydrogenase subunits characterized by SEQ ID Nos. 1, 3 and 5, i.e. the activity of the variants when used in combination in a heterotrimeric complex is at least 50% or 60%, preferably at least 70% or 80%, more preferably 85% or 90% and most preferably at least 95% or 98% of the activity of the fructose dehydrogenase characterized by the sequences according to SEQ ID Nos. 1, 3 and 5 when used in combination in a heterotrimeric complex.

The activity of the fructose dehydrogenase can be compared by incubating the fructose dehdrogenase (composed of the three subunits as defined above) with a suitable acceptor of the hydrogen atoms and measuring the amount of 5-ketofructose produced.

The amount of 5-ketofructose which is produced by the action of a fructose dehydrogenase, either for determining the activity of a variant or for determining the amount of 5-ketofructose produced by the methods of the present invention, can be determined by HPLC or by an enzymatic assay using a 5-ketofructose reductase, for example from *Tatumella morbirosei.* The 5-ketofructose reductase catalyzes the conversion of 5-ketofructose to fructose using NADPH as hydrogen donor. The increase of NADP⁺ can then be measured photometrically at 340 nm.

In the method of the present invention the acetic acid bacterium is incubated with a solution comprising a carbohydrate. The solution comprising a carbohydrate may be a solution which only comprises the carbohydrate dissolved in a suitable solvent such as water or it may be a culture medium which contains nutrients and salts in addition to said carbohydrate.

The term "carbohydrate" is intended to comprise biological molecules which are composed of carbon, oxygen and hydrogen atoms and includes monosaccharides, disaccharides, oligosaccharides and polysaccharides. If the carbohydrate is fructose, it may be used by the acetic acid bacterium used in the method to produce 5-ketofructose directly as the substrate for 5-ketofructose synthesis. Alternatively, at least a part of the carbohydrate may be enzymatically converted to fructose which then serves as the substrate for 5-ketofructose synthesis. The carbohydrate is preferably selected from the group consisting of fructose, glucose, sucrose and starch. If the acetic acid bacterium is genetically modified to express a fructose dehydrogenase and does not contain any additional genetic modifications affecting carbohydrate metabolism, the carbohydrate is fructose. If the acetic acid bacterium is genetically modified to express fructose dehydrogenase and glucose isomerase, the carbohydrate is glucose. If the acetic acid bacterium is genetically modified to express fructose dehydrogenase and invertase, possibly together with glucose isomerase, the carbohydrate is sucrose. If the acetic acid bacterium is genetically modified to express fructose dehydrogenase, glucose isomerase, amylase and pullulanase, the carbohydrate is starch.

The step of isolating the 5-ketofructose after it has been produced in the acetic acid bacterium means that the 5-ketofructose is separated from the acetic acid bacteria producing it and other compounds which may be present in the solution of 5-ketofructose, such as medium components. Typically, the 5-ketofructose is first separated from the acetic acid bacterium by centrifugation or filtration and is then isolated by any known method including adsorption on charcoal and treatment with cation or anion exchange resins. Suitable methods for isolating 5-ketofructose are described for example in US 3,206,375 and in Avigad and Englard (1965) J. Biol. Chem. 240(6): 2290-2296.

In one embodiment of the present invention, the method for producing 5-ketofructose comprises the steps of:
(a) incubating a purified first acetic acid bacterium which is genetically modified to express a fructose dehydrogenase with a solution comprising a carbohydrate; and
(b) isolating the 5-ketofructose.

If fructose is used as the carbohydrate which serves as a substrate for the fructose dehydrogenase, the concentration of fructose in the solution which is incubated with the purified first acetic acid bacterium is 30 to 1,000 g/l, preferably 80 to 800 g/l or 100 to 600 g/l, more preferably 150 to 500 g/l or 180 to 400 g/l and most preferably 200 to 250 g/l.

The term "purified acetic acid bacterium" is intended to mean that the acetic acid bacterium has been separated from the culture medium, before it is incubated with the carbohydrate. Typically, the acetic acid bacterium is separated from the culture medium by centrifugation, optionally washed with a suitable wash solution such as 0.9% sodium chloride solution or a buffer solution and then resuspended in the solution comprising the carbohydrate, before it is incubated with said solution. The incubation time depends on the cell density and the concentration of the substrate and can easily be determined by the skilled person. Optionally, the purified acetic acid bacterium can be fed with additional solution comprising the carbohydrate after it has been incubated in the initial solution comprising the carbohydrate for a period of time.

In one embodiment of the present invention, the method for producing 5-ketofructose comprises the steps of:
(a) culturing a first acetic acid bacterium which is genetically modified to express a fructose dehydrogenase in a culture medium comprising a carbohydrate; and
(b) isolating the 5-ketofructose.

The terms "cell culture" and "culturing of cells" refer to the maintenance and propagation of cells, preferably bacterial cells and more preferably of acetic acid bacteria.

The terms "medium", "cell culture medium" and "culture medium" are interchangeably used herein and refer to a solution containing nutrients which are required for growing bacterial cells. Typically, a cell culture medium for bacterial cells provides essential and non-essential amino acids, vitamins, energy sources such as a carbohydrate, and salts required by the cell for growth and/or survival. Most of the above components of the medium may be provided by adding yeast extract to the culture medium, thereby providing free amino acids, proteins, vitamins and nucleotides. The culture medium may further comprise a selection agent such as an antibiotic to select for the cells which have been genetically modified by the expression of an antibiotic resistance gene. If the introduced fructose dehydrogenase is expressed under the control of an inducible promoter, the medium may also comprise an inducing agent.

For culturing the bacterial cells different strategies are available, including batch culture, perfusion culture, continuous culture and fed-batch culture. Within the method of the present invention, preferably a fed-batch culture process is used. In fed-batch culture the culturing process is started with a certain volume of the basal medium and one or more feeds are fed at later time-point(s) of the culture process while no product is removed from the cell culture broth.

At the beginning of the culture or during the culture additional cells may be added. Further, a method with cell retention or cell reuse may be used, for example in sequential batches or repeated fed-batch methods.

The term "basal medium" is intended to refer to the medium which is used from the beginning of the cell culture process. The bacterial cells are inoculated into the basal medium and grown in this medium for a certain period until the feeding is started. The basal medium meets the definition of the culture medium as provided above. In the methods of the present invention the cells are grown in the basal medium for a period of 10 to 24 hours, preferably of 12 to 22 hours, more preferably of 15 to 20 hours and most preferably of 18 hours.

The pH of the medium may be adjusted to a pH of 5 to 6 using NaOH, KOH, Ca(OH)₂ or Mg(OH)₂ to avoid the acidification of the medium during fermentation. To avoid that the pH of the medium becomes alkaline, the pH may also be adjusted with HCl, H₂SO₄ or H₃PO₄.

To further increase the yield of 5-ketofructose, the cells may be cultured under phosphate limitation to limit the growth of the bacterial cells and to redirect the available carbon to product synthesis.

The feed is added to the cell culture after the cells have been cultured in the basal medium for a certain period. Within the methods of the present invention the feed is preferably a solution of the carbohydrate which is to be used as a substrate for the production of the 5-ketofructose, for example a solution of the carbohydrate in water or a cell culture medium comprising said carbohydrate. The feed may be added continuously or as a bolus at defined time points. Preferably, the feed is added continuously. In the methods of the present invention, the feed is added for a period of 20 to 50 hours, preferably of 23 to 40 hours, more preferably for 25 to 35 hours and most preferably of 30 hours. After the feeding has been stopped, the cells may be kept in culture until all the fructose in the cell culture medium has been consumed, for example for another 10 to 40 hours, preferably for another 14 to 35 hours, more preferably for another 20 to 30 hours and most preferably for another 24 hours.

If fructose is added to the culture medium as the carbohydrate which serves as a substrate for the fructose dehydrogenase, the concentration of fructose in the culture medium is 30 to 300 g/l, preferably 50 to 250 g/l or 70 to 220 g/l, more preferably 100 to 200 g/l and most preferably 130 to 190 g/l. If the cells are cultured in fed-batch mode, the concentration of the fructose in the culture medium as listed above is the sum of the fructose concentration present in the basal medium and the fructose concentration which was fed to the culture at a specific time-point.

If a fructose feed is used in the fed-batch method as the carbohydrate which serves as a substrate for the fructose dehydrogenase, the concentration of fructose in the solution which is fed to the bacterial cells in the fed-batch mode is 300 to 1,500 g/l, preferably 500 to 1,300 g/l, more preferably 700 to 1,200 g/l and most preferably it is 1,000 g/l.

Hence, in one embodiment the method for producing 5-ketofructose comprises the steps of:
(a) culturing a first acetic acid bacterium which is genetically modified to express a fructose dehydrogenase in a basal medium comprising 50 to 250 g/l fructose;
(b) feeding the first acetic acid bacterium with a solution comprising 300 to 1,500 g/l fructose and
(c) isolating the 5-ketofructose.

Using the above method, a product yield, defined as the ratio of the amount of 5-ketofructose produced to the amount of fructose added to the cells, is at least 80% or 82%, preferably at least 84% or 86%, more preferably at least 88% and most preferably at least 90%.

The acetic acid bacterium may also use glucose as carbohydrate to produce 5-ketofructose. In this case, the glucose has to be converted to fructose by a glucose isomerase and the fructose is then used as a substrate for the fructose dehydrogenase. The glucose isomerase can be added as an isolated enzyme to the solution comprising glucose. Alternatively, the first acetic acid bacterium may be genetically modified to express the glucose isomerase. For example, the first acetic acid bacterium may comprise a second expression vector comprising a nucleic acid sequence which encodes a glucose isomerase or the nucleic acid sequences encoding the glucose isomerase and the fructose dehydrogenase may both be present on the first expression vector. In a third alternative a second acetic acid bacterium which is genetically modified to express a glucose isomerase, preferably comprises an expression vector comprising a nucleic acid sequence encoding the glucose isomerase, may be used in a mixed culture with the first acetic acid bacterium.

Nucleic acid sequences encoding suitable glucose isomerases which can be used in the methods of the present invention are known to the skilled person and include nucleic acid sequences from *Bacillus coagulans* (UniProt entry AJO24439.1), E. coli K12 (UniProt entry b3565) and *Streptomyces griseus* (NCBI entry SGR_RS05195) and *Bifidobacterium adolescentis* (encoding the protein with NCBI entry WP_085379355).

Since the acetic acid bacterium may metabolize the glucose also to gluconate by the action of a glucose dehydrogenase, the acetic acid bacteria used together with glucose as the carbohydrate for the production of 5-ketofructose should lack activity of a membrane-bound and preferably of both a membrane-bound and a soluble glucose dehydrogenase. If the acetic acid bacterium is *Gluconobacter oxydans*, the membrane-bound glucose dehydrogenase is encoded by a nucleic acid sequence selected from the group consisting of:
a) a nucleic acid sequence comprising the sequence according to SEQ ID No. 9 or a fragment thereof;
b) a nucleic acid sequence encoding a protein comprising the amino acid sequence according to SEQ ID No. 10;
c) a nucleic acid sequence comprising a sequence which is at least 70 % identical to the sequence according to SEQ ID No. 9 or a fragment thereof;
d) a nucleic acid sequence hybridizing under stringent conditions with a complementary sequence of a nucleic acid sequence according to SEQ ID No. 9 or a fragment thereof; and
e) a nucleic acid sequence encoding the same membrane-bound glucose dehydrogenase as any of the nucleic acid sequences of (a) to (d) above, but differing from the nucleic acid sequences of (a) to (d) above due to the degeneracy of the genetic code.

The fragment of SEQ ID No. 9 has a length of at least 1,200 nucleotides, preferably of at least 1,300, 1,400 or 1,500 nucleotides, more preferably of at least 1,600, 1,700 or 1,800 nucleotides and most preferably of at least 1,900, 2,000, 2,100, 2,200, 2,300 or 2,400 nucleotides.

The sequence identity refers to the degree of the sequence identity over a length of at least 1,200 nucleotides, preferably of 1,300, 1,400, 1,500, 1,600, 1,700 or 1,800 nucleotides and more preferably of 1,900, 2,000, 2,100, 2,200, 2,300 or 2,400 nucleotides and most preferably the whole length of SEQ ID No. 9.

If the acetic acid bacterium is *Gluconobacter oxydans*,, the soluble glucose dehydrogenase is encoded by a nucleic acid sequence selected from the group consisting of:
a) a nucleic acid sequence comprising the sequence according to SEQ ID No. 11 or a fragment thereof;
b) a nucleic acid sequence encoding a protein comprising the amino acid sequence according to SEQ ID No. 12;
c) a nucleic acid sequence comprising a sequence which is at least 70 % identical to the sequence according to SEQ ID No. 11 or a fragment thereof;
d) a nucleic acid sequence hybridizing under stringent conditions with a complementary sequence of a nucleic acid sequence according to SEQ ID No. 11 or a fragment thereof; and
e) a nucleic acid sequence encoding the same soluble glucose dehydrogenase as any of the nucleic acid sequences of (a) to (d) above, but differing from the nucleic acid sequences of (a) to (d) above due to the degeneracy of the genetic code.

The fragment of SEQ ID No. 11 has a length of at least 400 nucleotides, preferably of at least 450, 500 or 550 nucleotides, more preferably of at least 600, 650 or 700 nucleotides and most preferably of at least 720, 740, 760, 770, 780 or 790 nucleotides.

The sequence identity refers to the degree of the sequence identity over a length of at least 400 nucleotides, preferably of 450, 500, 550, 600, 650 or 700 nucleotides and more preferably of 720, 740, 760, 770, 780 or 790 nucleotides and most preferably the whole length of SEQ ID No. 11.

Methods for providing acetic acid bacteria lacking the expression of one or more proteins are described in the prior art. For example, the article of Krajewski et al. (2010) Appl. Environm. Microbiol. 76(13): 4369-4376 describes the construction of mutant *Gluconobacter oxydans* strains lacking expression of a membrane-bound glucose dehydrogenase and/or a soluble glucose dehydrogenase.

Accordingly, in one embodiment the method for producing 5-ketofructose comprises the steps of:
(a) contacting a first acetic acid bacterium which is genetically modified to express a fructose dehydrogenase and which lacks expression of a membrane-bound glucose dehydrogenase with a solution comprising glucose and an isolated glucose isomerase; and
(b) isolating the 5-ketofructose.

Isolated glucose isomerase enzyme is commercially available, for example from Sigma Aldrich.

In one embodiment the method for producing 5-ketofructose comprises the steps of:
(a) contacting a first acetic acid bacterium which is genetically modified to express a fructose dehydrogenase and a glucose isomerase and which lacks expression of a membrane-bound glucose dehydrogenase with a solution comprising glucose; and
(b) isolating the 5-ketofructose.

In one embodiment the method for producing 5-ketofructose comprises the steps of:
(a) contacting a first acetic acid bacterium which is genetically modified to express a fructose dehydrogenase and which lacks expression of a membrane-bound glucose dehydrogenase and a second acetic acid bacterium which is genetically modified to express a glucose isomerase and which lacks expression of a membrane-bound glucose dehydrogenase with a solution comprising glucose; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase and a glucose isomerase and which lacks expression of a membrane-bound glucose dehydrogenase with a solution comprising glucose; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase and which lacks expression of a membrane-bound glucose dehydrogenase and a purified second acetic acid bacterium which is genetically modified to express a glucose isomerase and which lacks expression of a membrane-bound glucose dehydrogenase with a solution comprising glucose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and a glucose isomerase and which lacks expression of a membrane-bound glucose dehydrogenase in a culture medium comprising glucose; and
(b) isolating the 5-ketofructose.

The first acetic acid bacterium may comprise only one expression vector which comprises the nucleic acid sequences encoding both the fructose dehydrogenase and the glucose isomerase. Alternatively, the first acetic acid bacterium may comprise a first expression vector comprising a nucleic acid sequence encoding the fructose dehydrogenase and a second expression vector comprising a nucleic acid sequence encoding the glucose isomerase.

In one embodiment of the present invention, the method for producing 5-ketofructose comprises the steps of:
(a) culturing a first acetic acid bacterium which is genetically modified to express a fructose dehydrogenase and which lacks expression of a membrane-bound glucose dehydrogenase and a second acetic acid bacterium which is genetically modified to express a glucose isomerase and which lacks expression of a membrane-bound glucose dehydrogenase in a culture medium comprising glucose; and
(b) isolating the 5-ketofructose.

The acetic acid bacterium may also use sucrose as carbohydrate to produce 5-ketofructose. In this case, the sucrose has first to be cleaved into fructose and glucose by the action of an invertase and the fructose is then used as a substrate for the fructose dehydrogenase. The invertase can be added as an isolated enzyme to the solution comprising sucrose. Alternatively, the first acetic acid bacterium may be genetically modified to express the invertase. For example, it may comprise a second expression vector comprising a nucleic acid sequence which encodes an invertase or the nucleic acid sequences encoding the invertase and the fructose dehydrogenase may be present on the same expression vector. In a third alternative a second acetic acid bacterium may be used which is genetically modified to express an invertase, preferably comprises an expression vector comprising a nucleic acid sequence encoding the invertase.

The invertase may be encoded by a nucleic acid sequence selected from the group consisting of:
a) a nucleic acid sequence comprising the sequence according to SEQ ID No. 13 or a fragment thereof;
b) a nucleic acid sequence encoding a protein comprising the amino acid sequence according to SEQ ID No. 14;
c) a nucleic acid sequence comprising a sequence which is at least 70 % identical to the sequence according to SEQ ID No. 13 or a fragment thereof;
d) a nucleic acid sequence hybridizing under stringent conditions with a complementary sequence of a nucleic acid sequence according to SEQ ID No. 13 or a fragment thereof; and
e) a nucleic acid sequence encoding the same invertase as any of the nucleic acid sequences of (a) to (d) above, but differing from the nucleic acid sequences of (a) to (d) above due to the degeneracy of the genetic code.

The fragment of SEQ ID No. 13 has a length of at least 600 nucleotides, preferably of at least 650, 700 or 750 nucleotides, more preferably of at least 800, 850 or 900 nucleotides and most preferably of at least 950, 1,000, 1,050, 1,100 or 1,150 nucleotides.

The sequence identity refers to the degree of the sequence identity over a length of at least 600 nucleotides, preferably of 650, 700, 750, 800, 850 or 900 nucleotides and more preferably of 950, 1,000, 1,050, 1,100 or 1,150 nucleotides and most preferably the whole length of SEQ ID No. 13.

The variants of the sequence according to SEQ ID No. 13 as defined above encode a protein which has essentially the same enzymatic activity as the invertase encoded by the nucleic acid sequence according to SEQ ID No. 13, i.e. the activity of the variants is at least 50% or 60%, preferably at least 70% or 80%, more preferably at least 85% or 90% and most preferably at least 95% or 98% of the invertase encoded by the nucleic acid sequence according to SEQ ID No. 13.

Preferably, the glucose obtained by cleaving the sucrose by the action of the invertase is converted to fructose by the action of a glucose isomerase to increase the amount of fructose which can be used as a substrate for the fructose dehydrogenase. The glucose isomerase can be added as an isolated enzyme to the solution comprising sucrose. Alternatively, the first acetic acid bacterium may be genetically modified to express the glucose isomerase. For example, it may comprise a second expression vector comprising a nucleic acid sequence which encodes an invertase and a third expression vector comprising a nucleic acid sequence which encodes a glucose isomerase or the nucleic acid sequences encoding the invertase, the glucose isomerase and the fructose dehydrogenase may be present on the same expression vector. In a third alternative a second acetic acid bacterium may be used which is genetically modified to express an invertase, preferably comprises an expression vector comprising a nucleic acid sequence encoding the invertase and a third acetic acid bacterium may be used which is genetically modified to express a glucose isomerase, preferably comprises an expression vector comprising a nucleic acid sequence encoding the glucose isomerase.

The skilled person knows that any combinations of the above approaches may be used. Any of these combinations are is also within the scope of the present invention. For example, the first acetic acid bacterium may be genetically modified to express a fructose dehydrogenase and a glucose isomerase and may be contacted with the solution comprising sucrose together with a second acetic acid bacterium which is genetically modified to express an invertase.

Since the acetic acid bacterium may metabolize the glucose also to gluconate by the action of a glucose dehydrogenase, any of the acetic acid bacteria used together with sucrose as the carbohydrate for the production of 5-ketofructose and expressing a glucose isomerase should lack activity of a membrane-bound glucose dehydrogenase. Preferably, the acetic acid bacterium lacks expression of both a membrane-bound and a soluble glucose dehydrogenase. Nucleic acid sequences encoding a membrane-bound and a soluble glucose dehydrogenase have been described above.

Accordingly, in one embodiment the method for producing 5-ketofructose comprises the steps of:
(a) contacting a first acetic acid bacterium which is genetically modified to express a fructose dehydrogenase and which lacks expression of a membrane-bound glucose dehydrogenase with a solution comprising sucrose, an isolated glucose isomerase and an isolated invertase; and
(b) isolating the 5-ketofructose.

In one embodiment the method for producing 5-ketofructose comprises the steps of:
(a) contacting a first acetic acid bacterium which is genetically modified to express a fructose dehydrogenase and a glucose isomerase and which lacks expression of a membrane-bound glucose dehydrogenase with a solution comprising sucrose and an isolated invertase; and
(b) isolating the 5-ketofructose

In one embodiment the method for producing 5-ketofructose comprises the steps of:
(a) contacting a first acetic acid bacterium which is genetically modified to express a fructose dehydrogenase and an invertase and which lacks expression of a membrane-bound glucose dehydrogenase with a solution comprising sucrose and an isolated glucose isomerase; and
(b) isolating the 5-ketofructose.

In one embodiment the method for producing 5-ketofructose comprises the steps of:
(a) contacting a first acetic acid bacterium which is genetically modified to express a fructose dehydrogenase, an invertase and a glucose isomerase and which lacks expression of a membrane-bound glucose dehydrogenase with a solution comprising sucrose; and
(b) isolating the 5-ketofructose.

In one embodiment the method for producing 5-ketofructose comprises the steps of:
(a) contacting a first acetic acid bacterium which is genetically modified to express a fructose dehydrogenase and which lacks expression of a membrane-bound glucose dehydrogenase, a second acetic acid bacterium which is genetically modified to express a glucose isomerase and which lacks expression of a membrane-bound glucose dehydrogenase and a third acetic acid bacterium which is genetically modified to express an invertase and which lacks expression of a membrane-bound glucose dehydrogenase with a solution comprising sucrose; and
(b) isolating the 5-ketofructose.

In one embodiment the method for producing 5-ketofructose comprises the steps of:
(a) contacting a first acetic acid bacterium which is genetically modified to express a fructose dehydrogenase and a glucose isomerase and which lacks expression of a membrane-bound glucose dehydrogenase and a second acetic acid bacterium which is genetically modified to express an invertase and which lacks expression of a membrane-bound glucose dehydrogenase with a solution comprising sucrose; and
(b) isolating the 5-ketofructose.

In one embodiment the method for producing 5-ketofructose comprises the steps of:
(a) contacting a first acetic acid bacterium which is genetically modified to express a fructose dehydrogenase and an invertase and which lacks expression of a membrane-bound glucose dehydrogenase and a second acetic acid bacterium which is genetically modified to express a glucose isomerase and which lacks expression of a membrane-bound glucose dehydrogenase with a solution comprising sucrose; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase, an invertase and a glucose isomerase and which lacks expression of a membrane-bound glucose dehydrogenase with a solution comprising sucrose; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase and which lacks expression of a membrane-bound glucose dehydrogenase, a purified second acetic acid bacterium which is genetically modified to express a glucose isomerase and which lacks expression of a membrane-bound glucose dehydrogenase and a purified third acetic acid bacterium which is genetically modified to express an invertase and which lacks expression of a membrane-bound glucose dehydrogenase with a solution comprising sucrose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase, an invertase and a glucose isomerase and which lacks expression of a membrane-bound glucose dehydrogenase in a culture medium comprising sucrose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and an invertase and which lacks expression of a membrane-bound glucose dehydrogenase and a second acetic acid bacterium which is genetically modified to express a glucose isomerase and which lacks expression of a membrane-bound glucose dehydrogenase in a culture medium comprising sucrose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and a glucose isomerase and which lacks expression of a membrane-bound glucose dehydrogenase and a second acetic acid bacterium which is genetically modified to express an invertase and which lacks expression of a membrane-bound glucose dehydrogenase in a culture medium comprising sucrose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and which lacks expression of a membrane-bound glucose dehydrogenase and a second acetic acid bacterium which is genetically modified to express an invertase and a glucose isomerase and which lacks expression of a membrane-bound glucose dehydrogenase in a culture medium comprising sucrose; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and which lacks expression of a membrane-bound glucose dehydrogenase, a second acetic acid bacterium which is genetically modified to express a glucose isomerase and which lacks expression of a membrane-bound glucose dehydrogenase and a third acetic acid bacterium which is genetically modified to express an invertase and which lacks expression of a membrane-bound glucose dehydrogenase in a culture medium comprising sucrose; and
(b) isolating the 5-ketofructose.

The concentration of sucrose in the culture medium may be 10 to 250 g/l, preferably 30 to 220 g/l, more preferably 50 to 200 g/l and most preferably 100 to 150 g/l.

As discussed above, the bacterium may advantageously be cultured in the fed-batch mode wherein a solution comprising sucrose is fed to the cells after the cells have been cultured in basal medium comprising sucrose for a certain period of time.

The acetic acid bacterium may also use starch as carbohydrate to produce 5-ketofructose. In this case, the starch has first to be cleaved into glucose by an amylase and/or a glucoamylase and a pullulanase. The amylase cleaves the α-1,4 bonds within the starch and the pullulanase cleaves the α-1,6 bonds within the starch. The glucose is then converted to fructose by a glucose isomerase and the fructose is used as a substrate for the fructose dehydrogenase to produce 5-ketofructose.

The amylase, pullulanase and/or glucose isomerase can be added as isolated enzymes to the solution comprising starch. Alternatively, the first acetic acid bacterium may be genetically modified to express the amylase, pullulanase and/or glucose isomerase. For example, it may comprise a second expression vector comprising a nucleic acid sequence which encodes an amylase, a third expression vector comprising a nucleic acid sequence which encodes a pullulanase and a fourth expression vector comprising a nucleic acid sequence which encodes a glucose isomerase or the nucleic acid sequences encoding the amylase, the pullulanase, the glucose isomerase and the fructose dehydrogenase may be present on the first expression vector. In a third alternative a second acetic acid bacterium which is genetically modified to express an amylase, preferably comprises an expression vector comprising a nucleic acid sequence encoding the amylase, a third acetic acid bacterium which is genetically modified to express a pullulanase, preferably comprises an expression vector comprising a nucleic acid sequence encoding the pullulanase and/or a fourth acetic acid bacterium which is genetically modified to express a glucose isomerase, preferably comprises an expression vector comprising a nucleic acid sequence encoding the glucose isomerase, may be used.

The skilled person knows that any combinations of the above approaches may be used. Any of these combinations are is also within the scope of the present invention. For example, the first acetic acid bacterium may be genetically modified to express a fructose dehydrogenase and a glucose isomerase and may be contacted with the solution comprising starch together with a second acetic acid bacterium which is genetically modified to express an amylase and a pullulanase.

Since the acetic acid bacterium may metabolize the glucose also to gluconate by the action of a glucose dehydrogenase, any of the acetic acid bacteria used together with starch as the carbohydrate for the production of 5-ketofructose should lack expression of a membrane-bound glucose dehydrogenase. Preferably, the acetic acid bacterium lacks expression of both a membrane-bound and a soluble glucose dehydrogenase. Nucleic acid sequences encoding a membrane-bound and a soluble glucose dehydrogenase have been described above.

Amylases and glucoamylaseswhich can be used in the methods of the present invention are known to the skilled person and include the α-amylase from *Streptomyces griseus* (Cadenas et al. (1996) FEMS Microbiology Letters 137: 63-68), from *Streptococcus bovis* (Inokuma et al. (2015) Appl. Microbiol. Biotechnol. 99:1655-1663) or from *Geobacillus stearothermophilus* (Suzuki et al (2009) Appl. Microbiol. Biotechnol. 82:491-500) and the glucoamylase from *Rhizopus oryzae* (Inokuma et al. (2015) Appl. Microbiol. Biotechnol. 99:1655-1663)

Pullulanases which can be used in the methods of the present invention are known to the skilled person and include the pullulanase from *Klebsiella pneumoniae* (d'Enfert et al. (1987) EMBO J. 6(11):.3531 -3538), from *Geobacillus thermoleovorans* (Ayadi et al. (2008) Appl. Microbiol. Biotechnol. 78:473-481, from *Bacillus naganoensis* (Nie et al. (2013) PLoS ONE 8(10): e78416) and from *Bacillus acidopullulyticus* (Chen et al. (2014) J. Indust. Microbiol. Biotechnol. 41:1803-1810).

Accordingly, in one embodiment the method for producing 5-ketofructose comprises the steps of:
(a) contacting a first acetic acid bacterium which is genetically modified to express a fructose dehydrogenase and which lacks expression of a membrane-bound glucose dehydrogenase with a solution comprising starch, an isolated glucose isomerase, an isolated pullulanase and an isolated amylase; and
(b) isolating the 5-ketofructose

In one embodiment the method for producing 5-ketofructose comprises the steps of:
(a) contacting a first acetic acid bacterium which is genetically modified to express a fructose dehydrogenase and a glucose isomerase and which lacks expression of a membrane-bound glucose dehydrogenase with a solution comprising starch, an isolated amylase and an isolated pullulanase; and
(b) isolating the 5-ketofructose

In one embodiment the method for producing 5-ketofructose comprises the steps of:
(a) contacting a first acetic acid bacterium which is genetically modified to express a fructose dehydrogenase and an amylase and which lacks expression of a membrane-bound glucose dehydrogenase with a solution comprising starch, an isolated glucose isomerase and an isolated pullulanase; and
(b) isolating the 5-ketofructose.

In one embodiment the method for producing 5-ketofructose comprises the steps of:
(a) contacting a first acetic acid bacterium which is genetically modified to express a fructose dehydrogenase and a pullulanase and which lacks expression of a membrane-bound glucose dehydrogenase with a solution comprising starch, an isolated glucose isomerase and an isolated amylase; and
(b) isolating the 5-ketofructose.

Isolated amylases and pullulanases are available for example from Sigma Aldrich.

In one embodiment the method for producing 5-ketofructose comprises the steps of:
(a) contacting a first acetic acid bacterium which is genetically modified to express a fructose dehydrogenase, an amylase, a pullulanase and a glucose isomerase and which lacks expression of a membrane-bound glucose dehydrogenase with a solution comprising starch; and
(b) isolating the 5-ketofructose.

In one embodiment the method for producing 5-ketofructose comprises the steps of:
(a) contacting a first acetic acid bacterium which is genetically modified to express a fructose dehydrogenase and which lacks expression of a membrane-bound glucose dehydrogenase, a second acetic acid bacterium which is genetically modified to express a glucose isomerase and which lacks expression of a membrane-bound glucose dehydrogenase, a third acetic acid bacterium which is genetically modified to express an amylase and which lacks expression of a membrane-bound glucose dehydrogenase and a fourth acetic acid bacterium which is genetically modified to express a pullulanase with a solution comprising starch; and
(b) isolating the 5-ketofructose.

In one embodiment the method for producing 5-ketofructose comprises the steps of:
(a) contacting a first acetic acid bacterium which is genetically modified to express a fructose dehydrogenase and a glucose isomerase and which lacks expression of a membrane-bound glucose dehydrogenase and a second acetic acid bacterium which is genetically modified to express an amylase and a pullulanase and which lacks expression of a membrane-bound glucose dehydrogenase with a solution comprising starch; and
(b) isolating the 5-ketofructose.

In one embodiment the method for producing 5-ketofructose comprises the steps of:
(a) contacting a first acetic acid bacterium which is genetically modified to express a fructose dehydrogenase and an amylase and which lacks expression of a membrane-bound glucose dehydrogenase and a second acetic acid bacterium which is genetically modified to express a glucose isomerase and a pullulanase and which lacks expression of a membrane-bound glucose dehydrogenase with a solution comprising starch; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase, an amylase, a pullulanase and a glucose isomerase and which lacks expression of a membrane-bound glucose dehydrogenase with a solution comprising starch; and
(b) isolating the 5-ketofructose.

In one embodiment of the present invention, the method for producing 5-ketofructose comprises the steps of:
(a) incubating a purified first acetic acid bacterium which is genetically modified to express a fructose dehydrogenase and which lacks expression of a membrane-bound glucose dehydrogenase, a purified second acetic acid bacterium which is genetically modified to express a glucose isomerase and which lacks expression of a membrane-bound glucose dehydrogenase, a purified third acetic acid bacterium which is genetically modified to express an amylase and which lacks expression of a membrane-bound glucose dehydrogenase and a fourth acetic acid bacterium which is genetically modified to express a pullulanase with a solution comprising starch; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase, an amylase, a pullulanase and a glucose isomerase and which lacks expression of a membrane-bound glucose dehydrogenase in a culture medium comprising starch; and
(b) isolating the 5-ketofructose.

In one embodiment of the present invention, the method for producing 5-ketofructose comprises the steps of:
(a) culturing a first acetic acid bacterium which is genetically modified to express a fructose dehydrogenase and which lacks expression of a membrane-bound glucose dehydrogenase, a second acetic acid bacterium which is genetically modified to express a glucose isomerase and which lacks expression of a membrane-bound glucose dehydrogenase, a third acetic acid bacterium which is genetically modified to express an amylase and which lacks expression of a membrane-bound glucose dehydrogenase and a fourth acetic acid bacterium which is genetically modified to express a pullulanase and which lacks expression of a membrane-bound glucose dehydrogenase in a culture medium comprising starch; and
(b) isolating the 5-ketofructose.

As discussed above, the bacterium may advantageously be cultured in the fed-batch mode wherein a solution comprising starch is fed to the cells after the cells have been cultured in basal medium comprising glucose, fructose or starch for a certain period of time.

The acetic acid bacterium may additionally comprise a genetic modification enabling the export of the proteins to the outside of the bacterial cell. The genetic modification may be such that it causes a leaky outer membrane. Genetic modifications causing a leaky outer membrane include the deletion of any of the genes selected from the group consisting of *tolA*, *tolB*, *tolQRA*, *rfa*, *pal* und *lpp* (see Lazzaroni et al. (1999) FEMS Microbiol. Lett 177: 191-197). Preferably, the genetic modification enabling the export of proteins and causing a leaky outer membrane is a deletion of the *tolB* gene.

The *tolB* gene may have a nucleic acid sequence selected from the group consisting of:
a) a nucleic acid sequence comprising the sequence according to SEQ ID No. 15 or a fragment thereof;
b) a nucleic acid sequence encoding a protein comprising the amino acid sequence according to SEQ ID No. 16;
c) a nucleic acid sequence comprising a sequence which is at least 70 % identical to the sequence according to SEQ ID No. 15 or a fragment thereof;
d) a nucleic acid sequence hybridizing under stringent conditions with a complementary sequence of a nucleic acid sequence according to SEQ ID No. 15 or a fragment thereof; and
e) a nucleic acid sequence encoding the same TolB protein as any of the nucleic acid sequences of (a) to (d) above, but differing from the nucleic acid sequences of (a) to (d) above due to the degeneracy of the genetic code.

The fragment of SEQ ID No. 15 has a length of at least 650 nucleotides, preferably of at least 700, 750 or 800 nucleotides, more preferably of at least 850, 900 or 950 nucleotides and most preferably of at least 1,000, 1,050, 1,100 1,150, 1,200 or 1,250 nucleotides.

The sequence identity refers to the degree of the sequence identity over a length of at least 650 nucleotides, preferably of 700, 750, 800, 850, 900 or 950 nucleotides and more preferably of 1,000, 1,050, 1,100, 1,150, 1,200 or 1,250 nucleotides and most preferably the whole length of SEQ ID No. 15.

The article of Kosciow et al. (2016) J. Ind. Microbiol. Biotechnol. 43: 989-999 describes the construction of a mutant *Gluconobacter oxydans* strain with a deletion of the *tolB* gene.

The 5-ketofructose produced by any of the methods described above can be used to prepare a sweetener composition. A sweetener composition is a composition which is used to provide sweet taste to food such as desserts, fruit preparations, marmalade, chocolate and chocolate products, cakes, bread, joghurt, cereals, pudding, softdrinks and sauces. The sweetener composition can be added to the food before consumption or already during preparation of the food product.

The sweetener composition may be in any form which is suitable for the intended use. These forms include, but are not limited to, a solution, drops, gels, crystals, powder, granules and pills.

The sweetener composition may comprise only the 5-ketofructose and no further ingredients. Alternatively, the sweetener composition may comprise one or more additional compounds which are compatible with food, such as extenders, binders, stabilizers, preservers, flavourings and desiccants.

For oral consumption the 5-ketofructose may be mixed with acceptable excipients or carriers such as, but not being limited to, sodium hydrogen carbonate, lactose, tartaric acid, calcium carbonate, calcium phosphate, microcrystalline cellulose, dextrose, magnesium stearate, sucrose, plant gums, methylcellulose, povidone, carboxymethylcellulose and hydroxypropylmethylcellulose.

### EXAMPLES

### Example 1: Comparison of 5-ketofructose production in wild-type strain and recombinant strain expressing fructose dehydrogenase

The *Gluconobacter oxydans* wild-type strain 621H ΔhsdR and a genetically modified *Gluconobacter oxydans* strain transformed with plasmid pBBR1p264-FDH Strep (see SEQ ID No. 17) were cultured in a medium comprising 6 g/l yeast extract, 100 mM fructose and 100 mM potassium phosphate buffer, pH 6.8 at a temperature of 30°C for 72 hours. At different time-points samples were taken and the amount of fructose and 5-ketofructose was determined by HPLC. To this end, the samples were centrifuged, the supernatant was filtered and diluted with water containing 5 mM H₂SO₄.. The samples were analyzed with a Knauer HPLC system using the column Aminex-HPX87H 300 x 7.8 mm (Bio-Rad) and the following conditions: mobile phase: 5 mM H₂SO₄, temperature 21ºC for fructose determination and 65°C for 5-ketofructose determination, flow rate 0.3 ml/min, injection volume 20 µl. Detection was performed by using the Knauer UV-detector 2600 and RI-detector 101. For analysis the software ChromeGate Client (version 3.1.7) was used.

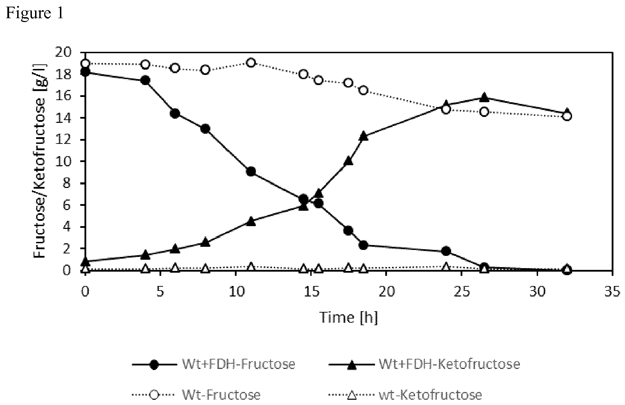

The results of this experiment are shown in Figure 1. The wild-type strain does not convert the fructose to 5-ketofructose, but only to biomass. The conversion of fructose is slow so that after 32 hours only a quarter of the fructose is degraded. In contrast, the *Glucose oxydans* strain converts 18 g/l fructose to 16 g/l 5-ketofructose within 27 hours.

### Example 2: Production of 5-ketofructose using fructose as carbohydrate

The *Gluconobacter oxydans* strain transformed with plasmid pBBR1p264-FDH Strep (SEQ ID No. 17) was cultured in a complex medium comprising 150 g/l fructose, 5 g/l yeast extract, 1 g/l potassium dihydrogen phosphate, 1 g/l ammonium sulfate, 2.5 g/l magnesium-heptahydrate and 50 µg/ml kanamycin. For the preculture 80 g/l mannitol were used instead of fructose as the carbon source. The pH in the medium of the pre culture and the main culture was set to pH 6 and was not regulated.

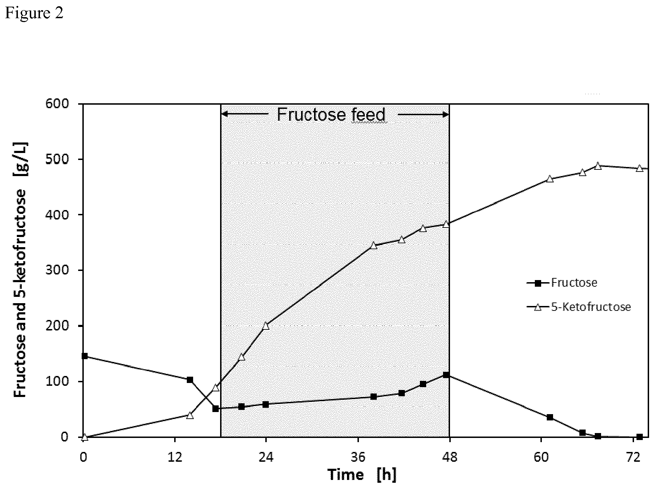

In the fed-batch phase a solution of 1,035 g/l fructose in water was fed and the pH was regulated to pH 5.5 with 3 mM KOH and 2 M HCl. In both the batch and the fed-batch phase the temperature was 30ºC, the dissolved oxygen concentration pO₂ was ≥ 30% and the gassing was 1 L/min. In the batch phase the maximum stirrer speed was 1,308 rpm. In the fed-batch phase the cells were fed with 850 ml of a 1,035 g/l fructose solution at a feed rate of 26 ml/h and the maximum stirrer speed was 1,500 rpm. At different time-points samples were taken and the amount of fructose and 5-ketofructose was determined by HPLC as described in Example 1. The results of this experiment are shown in Figure 2.

After 18 h in the batch phase a feed with 1,035 g/l fructose was started. After 48 hours the feed was stopped and the culture was continued for another 22 hours until the fructose was completely consumed. From the 562 g/l fructose introduced 489 g/l 5-ketofructose were obtained, which corresponds to a product yield of 87%.

### Example 3: Production of 5-ketofructose using sucrose as carbohydrate

For producing 5-ketofructose from sucrose a mixed culture of two genetically modified *Gluconobacter oxydans* strains was used. The first strain was *Gluconobacter oxydans* 621H Δhsd ΔtolB containing the plasmid pBBR1p264-sacC which expresses an invertase and has a deletion of the *tolB* gene leading to a leaky outer membrane (SEQ ID No. 18). The second strain was a genetically modified *Gluconobacter oxydans* strain transformed with plasmid pBBR1p264-FDH Strep and therefore overexpressing a fructose dehydrogenase.

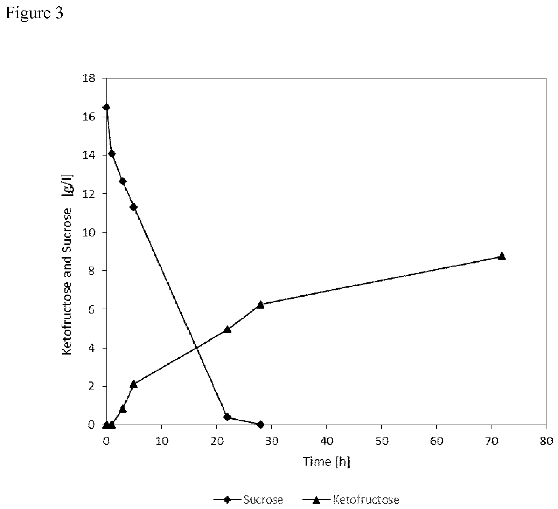

In a preculture on 9 g/l glucose both strains were cultured at 30ºC and 180 rpm to an OD₆₀₀ of 0.6. Then the two strains were combined and 17 g/l sucrose was added. At different time-points samples were taken and the amount of fructose and 5-ketofructose was determined by HPLC as described in Example 1. The results of this experiment are shown in Figure 3.

In the mixed culture the fructose obtained by cleaving the sucrose was converted almost completely to 8.7 g/l 5-ketofructose. Since in this experiment the strains did not comprise a deletion of glucose dehydrogenase and since no glucose isomerase was used to convert glucose to fructose, the glucose from the preculture and from the cleavage of sucrose was converted to 2-ketogluconate via gluconate and to acetate. Nevertheless, this example shows that sucrose can be used as a substrate for 5-ketofructose production.

Some embodiments of the present invention relate to:
1. Method for producing 5-ketofructose in an acetic acid bacterium, comprising the steps of:
   a) contacting a first acetic acid bacterium which is genetically modified to express a fructose dehydrogenase with a solution comprising a carbohydrate; and
   b) isolating the 5-ketofructose.
2. Method of item 1, wherein the first acetic acid bacterium contains a first expression vector comprising a nucleic acid sequence which encodes a fructose dehydrogenase.
3. Method of item 1 or 2, wherein the acetic acid bacterium is from the genus Gluconobacter.
4. Method of any one of the preceding items, wherein the acetic acid bacterium is *Gluconobacter oxydans.*
5. Method of any one of the preceding items, wherein the expression vector further comprises a promoter region from Gluconobacter.
6. Method of claim 5, wherein the promoter region is from a gene encoding a ribosomal protein.
7. Method of any one of the preceding items, wherein step (a) comprises incubating a purified acetic acid bacterium with a solution comprising said carbohydrate.
8. Method of any one of items 1 to 6, wherein step (a) comprises culturing the acetic acid bacterium in a culture medium comprising said carbohydrate.
9. Method of item 8, wherein the acetic acid bacterium is cultured in fed-batch mode.
10. Method of any one of the preceding items, wherein the carbohydrate is fructose.
11. Method of item 9, wherein the carbohydrate is fructose and the concentration of fructose in the culture medium is 30 to 300 g/l.
12. Method of any one of items 9 or 11, wherein the carbohydrate is fructose and the cells are fed with a solution comprising 300 to 1,500 g/l fructose.
13. Method of any one of items 1 to 9, wherein the carbohydrate is glucose and the glucose is converted to fructose by the action of a glucose isomerase and wherein the acetic acid bacterium lacks expression of a membrane-bound glucose dehydrogenase.
14. Method of item 13, wherein an isolated glucose isomerase is added to the solution comprising glucose.
15. Method of item 13, wherein the first acetic acid bacterium is genetically modified to express a glucose isomerase.
16. Method of claim 15, wherein the first acetic acid bacterium comprises a second expression vector comprising a nucleic acid sequence which encodes a glucose isomerase.
17. Method of item 13, wherein a second acetic acid bacterium genetically modified to express a glucose isomerase is contacted with said solution comprising glucose.
18. Method of item 17, wherein the second acetic acid bacterium contains an expression vector comprising a nucleic acid sequence which encodes a glucose isomerase
19. Method of any one of items 1 to 9, wherein the carbohydrate is sucrose and the sucrose is converted to fructose by the action of a glucose isomerase and an invertase and wherein the acetic acid bacterium lacks expression of a membrane-bound glucose dehydrogenase.
20. Method of item 19, wherein an isolated glucose isomerase and/or invertase is added to the solution comprising sucrose.
21. Method of item 19, wherein the first acetic acid bacterium is genetically modified to express a glucose isomerase and/or an invertase.
22. Method of item 21, wherein the first acetic acid bacterium comprises a second expression vector comprising a nucleic acid sequence which encodes a glucose isomerase and/or a third expression vector comprising a nucleic acid sequence which encodes an invertase.
23. Method of item 19, wherein a second acetic acid bacterium genetically modified to express a glucose isomerase and/or an invertase is contacted with said solution comprising sucrose.
24. Method of item 23, wherein the second acetic acid bacterium contains an expression vector comprising a nucleic acid sequence which encodes a glucose isomerase and/or a nucleic acid sequence which encodes an invertase.
25. Method of item 19, wherein a second acetic acid bacterium genetically modified to express a glucose isomerase and a third acetic acid bacterium genetically modified to express an invertase are contacted with said solution comprising sucrose.
26. Method of item 25, wherein the second acetic acid bacterium contains an expression vector comprising a nucleic acid sequence which encodes a glucose isomerase and the third acetic acid bacterium contains an expression vector comprising a nucleic acid sequence which encodes an invertase.
27. Method of any one of items 1 to 9 wherein the carbohydrate is starch and the starch is converted to fructose by the action of an amylase, a pullulanase and a glucose isomerase and wherein the acetic acid bacterium lacks expression of a membrane-bound glucose dehydrogenase.
28. Method of item 27, wherein an isolated glucose isomerase and/or amylase and/or pullulanase is added to the solution comprising starch.
29. Method of item 27, wherein the first acetic acid bacterium is genetically modified to express a glucose isomerase, an amylase and/or a pullulanase.
30. Method of item 29, wherein the first acetic acid bacterium comprises a second expression vector comprising a nucleic acid sequence which encodes a glucose isomerase and/or a third expression vector comprising a nucleic acid sequence which encodes an amylase and/or a fourth expression vector comprising a nucleic acid sequence which encodes a pullulanase.
31. Method of item 27, wherein a second acetic acid bacterium genetically modified to express a glucose isomerase and/or a third acetic acid bacterium genetically modified to express an amylase and/or a fourth acetic acid bacterium genetically modified to express a pullulanase is contacted with said solution comprising starch.
32. Method of item 31, wherein the second acetic acid bacterium contains an expression vector comprising a nucleic acid sequence which encodes a glucose isomerase, the third acetic acid bacterium contains an expression vector comprising a nucleic acid sequence which encodes an amylase and the fourth acetic acid bacterium contains an expression vector comprising a nucleic acid sequence which encodes a pullulanase.
33. Method of any one of items 13 to 32, wherein the acetic acid bacterium lacks expression of both a membrane-bound and a soluble glucose dehydrogenase.
34. Method of any one of items 13 to 33, wherein the acetic acid bacterium comprises a genetic modification causing a leaky outer membrane.
35. Method of item 34, wherein the genetic modification is a deletion of the *tolB* gene.
36. Method for producing a sweetener composition, comprising producing 5-ketofructose by the method of any one of items 1 to 35 and preparing a sweetener composition comprising 5-ketofructose.
37. Method of item 36, wherein the sweetener composition is a powder, solution, granule or pill.
38. Use of an acetic acid bacterium which is genetically modified to express a fructose dehydrogenase for producing 5-ketofructose.
39. Use of item 38, wherein the acetic acid bacterium contains a first expression vector comprising a nucleic acid sequence which encodes a fructose dehydrogenase.
40. Use of item 38 or 39, wherein the acetic acid bacterium is *Gluconobacter oxydans.*
41. Use of any one of items 38 to 40, wherein the acetic acid bacterium is further genetically modified to express a glucose isomerase.
42. Use of item 41, wherein the acetic acid bacterium comprises a second expression vector comprising a nucleic acid sequence which encodes a glucose isomerase.
43. Use of item 41 or 42, wherein the acetic acid bacterium is further genetically modified to express an invertase.
44. Use of item 43, wherein the acetic acid bacterium further comprises a third expression vector comprising a nucleic acid sequence which encodes an invertase.
45. Use of item 41 or 42, wherein the acetic acid bacterium is further genetically modified to express an amylase and a pullulanase.
46. Use of item 45, wherein the acetic acid bacterium further comprises a third expression vector comprising a nucleic acid sequence which encodes an amylase and a fourth expression vector comprising a nucleic acid sequence which encodes a pullulanase.
47. *Gluconobacter oxydans* cell containing an expression vector comprising a first nucleic acid sequence which encodes a fructose dehydrogenase and a second nucleic acid sequence which encodes a glucose isomerase.
48. *Gluconobacter oxydans* cell of item 47, wherein the expression vector further comprises a third nucleic acid sequence which encodes an invertase.
49. *Gluconobacter oxydans* cell of item 47, wherein the expression vector further comprises a third nucleic acid sequence which encodes an amylase and/or a fourth nucleic acid sequence which encodes a pullulanase.
50. *Gluconobacter oxydans* cell containing a first expression vector comprising a nucleic acid sequence which encodes a fructose dehydrogenase and a second expression vector comprising a nucleic acid sequence which encodes a glucose isomerase.
51. *Gluconobacter oxydans* cell of item 50 further containing a third expression vector comprising a nucleic acid sequence which encodes an invertase.
52. *Gluconobacter oxydans* cell of item 50 further containing a third expression vector comprising a nucleic acid sequence which encodes an amylase and/or a fourth expression vector comprising a nucleic acid sequence which encodes a pullulanase.
53. *Gluconobacter oxydans* cell of any one of items 47 to 52, lacking expression of a membrane-bound glucose dehydrogenase.
54. *Gluconobacter oxydans* cell of item 53, lacking expression of both a membrane-bound or a soluble glucose dehydrogenase.
55. *Gluconobacter oxydans* cell of any one of items 47 to 54, further comprising a mutation causing a leaky outer membrane.
56. *Gluconobacter oxydans* cell of item 55, wherein the mutation is a deletion of the *tolB* gene.

## Claims

1. Method for producing 5-ketofructose in an acetic acid bacterium, comprising the steps of:
a) contacting a first acetic acid bacterium which is genetically modified to express a fructose dehydrogenase with a solution comprising a carbohydrate; and
b) isolating the 5-ketofructose.

2. Method of claim 1, wherein the acetic acid bacterium is *Gluconobacter oxydans.*

3. Method of claim 1 or 2, wherein step (a) comprises culturing the acetic acid bacterium in a culture medium comprising said carbohydrate.

4. Method of any one of the preceding claims, wherein the expression vector further comprises a promoter region from Gluconobacter.

5. Method of any one of the preceding claims, wherein the carbohydrate is fructose.

6. Method of claim 5, wherein the acetic acid bacterium is cultured in fed-batch mode in a culture medium comprising fructose.

7. Method of claim 6, wherein the culture medium comprises 50 to 250 g/l fructose.

8. Method of any one of claims 1 to 4, wherein the carbohydrate is glucose and the glucose is converted to fructose by the action of a glucose isomerase, and wherein the acetic acid bacterium lacks expression of a membrane-bound glucose dehydrogenase.

9. Method of any one of claims 1 to 4, wherein the carbohydrate is sucrose and the sucrose is converted to fructose by the action of a glucose isomerase and an invertase and wherein the acetic acid bacterium lacks expression of a membrane-bound glucose dehydrogenase.

10. Method of any one of claims 1 to 4, wherein the carbohydrate is starch and the starch is converted to fructose by the action of an amylase, a pullulanase and a glucose isomerase and wherein the acetic acid bacterium lacks expression of a membrane-bound glucose dehydrogenase.

11. Method for producing a sweetener composition, comprising performing the method of any one of claims 1 to 10 and preparing a sweetener composition comprising 5-ketofructose.

12. Use of an acetic acid bacterium which is genetically modified to express a fructose dehydrogenase for producing 5-ketofructose.

13. *Gluconobacter oxydans* cell containing:
(a) an expression vector comprising a nucleic acid sequence which encodes a fructose dehydrogenase and a glucose isomerase or
(b) a first expression vector comprising a nucleic acid sequence which encodes a fructose dehydrogenase and a second expression vector comprising a nucleic acid sequence which encodes a glucose isomerase.

14. *Gluconobacter oxydans* cell of claim 13, wherein the expression vector of (a) further comprises a nucleic acid sequence which encodes an invertase or (b) further containing a third expression vector comprising a nucleic acid sequence which encodes an invertase.

15. *Gluconobacter oxydans* cell of claim 13, wherein the expression vector of (a) further comprises a nucleic acid sequence which encodes an amylase and a pullulanase or (b) further containing a third expression vector comprising a nucleic acid sequence which encodes an amylase and a fourth expression vector comprising a nucleic acid sequence which encodes a pullulanase.


SEQUENCE LISTING

<110>  RWTH Aachen
       Forschungszentrum Jülich
       Rheinische Friedrich-Wilhelms-Universität Bonn

<120>  Methods of producing 5-ketofructose

<130>  T08117EP/DB

<160>  18

<170>  PatentIn version 3.5

<210>  1
<211>  552
<212>  DNA
<213>  Gluconobacter japonicus

<400>  1
atggaaaaaa tagctgattc cggccctgtt caaatctttc tttcgcgtag aaagcttctg      60

gctttttccg gtgcctgcct aacagtcgca gcaattggaa caccatcgaa aggatctact     120

caggacgttg tggcattaaa tcgcgatagc atttcggatt ttatgcagct ttctgctttt     180

gccaccggtc acaaaaatct ggatctcaat atcggatcag cacttctctt ggcatttgaa     240

gctcagaagc atgatttttc tgctcaaata aaggctctcc gcgaacatat tactaaaaat     300

aattatcagg atgttgaagc actggatgca gccatgaaag atgaccccct gcatcccacg     360

ctcctccaga ttatccgggc gtggtattca ggtgtcatcg aagacggaac aaacgctaaa     420

gtttacgctt tcgaaaaagc actcatgtat cagccgtccc gcgatgtcgt ggtcattccg     480

acatatgctc ataacgggcc caattactgg gtctcggaac ccgcatccgt cgatgtcatg     540

ccggcatttt aa                                                         552

<210>  2
<211>  183
<212>  PRT
<213>  Gluconobacter japonicus

<400>  2

Met Glu Lys Ile Ala Asp Ser Gly Pro Val Gln Ile Phe Leu Ser Arg
1               5                   10                  15

Arg Lys Leu Leu Ala Phe Ser Gly Ala Cys Leu Thr Val Ala Ala Ile
            20                  25                  30

Gly Thr Pro Ser Lys Gly Ser Thr Gln Asp Val Val Ala Leu Asn Arg
        35                  40                  45

Asp Ser Ile Ser Asp Phe Met Gln Leu Ser Ala Phe Ala Thr Gly His
    50                  55                  60

Lys Asn Leu Asp Leu Asn Ile Gly Ser Ala Leu Leu Leu Ala Phe Glu
65                  70                  75                  80

Ala Gln Lys His Asp Phe Ser Ala Gln Ile Lys Ala Leu Arg Glu His
                85                  90                  95

Ile Thr Lys Asn Asn Tyr Gln Asp Val Glu Ala Leu Asp Ala Ala Met
            100                 105                 110

Lys Asp Asp Pro Leu His Pro Thr Leu Leu Gln Ile Ile Arg Ala Trp
        115                 120                 125

Tyr Ser Gly Val Ile Glu Asp Gly Thr Asn Ala Lys Val Tyr Ala Phe
    130                 135                 140

Glu Lys Ala Leu Met Tyr Gln Pro Ser Arg Asp Val Val Val Ile Pro
145                 150                 155                 160

Thr Tyr Ala His Asn Gly Pro Asn Tyr Trp Val Ser Glu Pro Ala Ser
                165                 170                 175

Val Asp Val Met Pro Ala Phe
            180

<210>  3
<211>  1461
<212>  DNA
<213>  Gluconobacter japonicus

<400>  3
atgcgatatt ttcgccctct gtccgccaca gccatgacaa ccgttctgct tctcgcaggc      60

acgaacgtac gggcgcaacc gacagaacca acacctgctt cagcccatag cccctccatc     120

agccgcggtc attatctggc aattgccgcc gattgtgcgg cctgccatac caatgggcgg     180

aacggtcagt tccttgctgg cggctatgcc atttcttccc caatgggaaa tatttactca     240

accaatatta cgccctcgaa gacgcacggt atcggcaact atactctgga gcagttttct     300

aaagcacttc ggcatggtat ccgcgccgat ggcgctcaac tgtatcccgc catgccttat     360

gacgcttaca atcgtctgac ggatgaagac gtcaaatcgc tctacgctta catcatgact     420

gaagtaaaac ccgtagatgc accttctccc aagacgcagc tccccttccc gttttcaatc     480

cgtgcatcac tcggtatttg gaaaattgcg gcaaggatcg aaggcaaacc ctatgtcttt     540

gatcatacgc acaatgacga ctggaaccgt ggtcgctatc tggtcgatga gctagcccat     600

tgcggtgaat gtcacacacc gcgtaacttt cttctggctc ccaatcagtc cgcttatctt     660

gctggtgcgg acataggatc atggcgtgca ccaaatatta ccaatgaccc tcaaagcggc     720

attggcagtt ggtcggatca ggatctcttc caatatctga agactggaaa aaccgcacat 780

gctcgtgcag caggcccaat ggcagaagca atcgagcaca gcctacaata tcttccggat 840

gccgatattt ctgccataat tacatatctg cgttcggttc cggcaaaggc agaaagcggt 900

caaacagtcg ctaattttga gcatgccggc cgcccatccg gttacagtgt tgctgacgct 960

aactcccgtc gcaccaacag tactctaaca aaaacaaccg atggtgctgc tctctacgag 1020

gctgtgtgcg ccagttgcca tcaatctgac ggtaaagggt ccaaagacgg ttattatccg 1080

tccctcgtag gaaatacgac gaccgggcag ctcaatccaa atgatctgat cgccagtatt 1140

ctttacggtg tggaccgcac aacggataac cacgaaatcc tgatgccggc ttttggtcca 1200

gactctctcg tacagcccct gacagatgag cagatcgcca caatcgcgga ctatgttcta 1260

agccactttg gcaatgctca ggccacagtc tcggcagatg cggtcaaaca ggtgcgggcg 1320

ggcggaaagc aagttcctct cgccaggctc gccagtccgg gcgtgatgct gcttctcgga 1380

gctggtggca tcctgggagc cattctggtg gtaggcggcc tctggtggct gatcagccgt 1440

cgcaaaaagc gttccgctta a 1461

<210> 4
<211> 486
<212> PRT
<213> Gluconobacter japonicus

<400> 4

Met Arg Tyr Phe Arg Pro Leu Ser Ala Thr Ala Met Thr Thr Val Leu
1               5                   10                  15

Leu Leu Ala Gly Thr Asn Val Arg Ala Gln Pro Thr Glu Pro Thr Pro
            20                  25                  30

Ala Ser Ala His Ser Pro Ser Ile Ser Arg Gly His Tyr Leu Ala Ile
        35                  40                  45

Ala Ala Asp Cys Ala Ala Cys His Thr Asn Gly Arg Asn Gly Gln Phe
    50                  55                  60

Leu Ala Gly Gly Tyr Ala Ile Ser Ser Pro Met Gly Asn Ile Tyr Ser
65                  70                  75                  80

Thr Asn Ile Thr Pro Ser Lys Thr His Gly Ile Gly Asn Tyr Thr Leu
                85                  90                  95

Glu Gln Phe Ser Lys Ala Leu Arg His Gly Ile Arg Ala Asp Gly Ala
            100                 105                 110

Gln Leu Tyr Pro Ala Met Pro Tyr Asp Ala Tyr Asn Arg Leu Thr Asp

115                 120                 125

Glu Asp Val Lys Ser Leu Tyr Ala Tyr Ile Met Thr Glu Val Lys Pro
    130                 135                 140

Val Asp Ala Pro Ser Pro Lys Thr Gln Leu Pro Phe Pro Phe Ser Ile
145                 150                 155                 160

Arg Ala Ser Leu Gly Ile Trp Lys Ile Ala Ala Arg Ile Glu Gly Lys
                165                 170                 175

Pro Tyr Val Phe Asp His Thr His Asn Asp Asp Trp Asn Arg Gly Arg
            180                 185                 190

Tyr Leu Val Asp Glu Leu Ala His Cys Gly Glu Cys His Thr Pro Arg
        195                 200                 205

Asn Phe Leu Leu Ala Pro Asn Gln Ser Ala Tyr Leu Ala Gly Ala Asp
    210                 215                 220

Ile Gly Ser Trp Arg Ala Pro Asn Ile Thr Asn Asp Pro Gln Ser Gly
225                 230                 235                 240

Ile Gly Ser Trp Ser Asp Gln Asp Leu Phe Gln Tyr Leu Lys Thr Gly
                245                 250                 255

Lys Thr Ala His Ala Arg Ala Ala Gly Pro Met Ala Glu Ala Ile Glu
            260                 265                 270

His Ser Leu Gln Tyr Leu Pro Asp Ala Asp Ile Ser Ala Ile Ile Thr
        275                 280                 285

Tyr Leu Arg Ser Val Pro Ala Lys Ala Glu Ser Gly Gln Thr Val Ala
    290                 295                 300

Asn Phe Glu His Ala Gly Arg Pro Ser Gly Tyr Ser Val Ala Asp Ala
305                 310                 315                 320

Asn Ser Arg Arg Thr Asn Ser Thr Leu Thr Lys Thr Thr Asp Gly Ala
                325                 330                 335

Ala Leu Tyr Glu Ala Val Cys Ala Ser Cys His Gln Ser Asp Gly Lys
            340                 345                 350

Gly Ser Lys Asp Gly Tyr Tyr Pro Ser Leu Val Gly Asn Thr Thr Thr
        355                 360                 365

Gly Gln Leu Asn Pro Asn Asp Leu Ile Ala Ser Ile Leu Tyr Gly Val
    370                 375                 380

Asp Arg Thr Thr Asp Asn His Glu Ile Leu Met Pro Ala Phe Gly Pro
385                 390                 395                 400

Asp Ser Leu Val Gln Pro Leu Thr Asp Glu Gln Ile Ala Thr Ile Ala
                405                 410                 415

Asp Tyr Val Leu Ser His Phe Gly Asn Ala Gln Ala Thr Val Ser Ala
            420                 425                 430

Asp Ala Val Lys Gln Val Arg Ala Gly Gly Lys Gln Val Pro Leu Ala
        435                 440                 445

Arg Leu Ala Ser Pro Gly Val Met Leu Leu Leu Gly Ala Gly Gly Ile
    450                 455                 460

Leu Gly Ala Ile Leu Val Val Gly Gly Leu Trp Trp Leu Ile Ser Arg
465                 470                 475                 480

Arg Lys Lys Arg Ser Ala
                485

<210>  5
<211>  1635
<212>  DNA
<213>  Gluconobacter japonicus

<400>  5
atgtctaatg aaacgctttc ggctgatgtc gtcatcattg gagcaggaat ctgcggctcc      60

ctgctcgccc acaaactcgt ccgcaacgga ctttctgtgc tgctcctgga tgcaggtccc     120

cgccgggacc gctcccagat cgtagaaaac tggcgcaaca tgccgcctga caacaagtcc     180

cagtatgatt atgcaacacc ttaccccagc gtaccttggg ccccgcatac caactacttc     240

ccagacaata actacctgat tgttaaaggc ccggaccgga cggcttacaa gcaaggaatc     300

atcaaaggcg tcgggggtac aacctggcac tgggcggctt caagctggcg gtacctgccc     360

aacgatttca agctgcattc cacatatggc gtgggccgcg attacgccat gagttacgac     420

gaacttgagc cgtactatta cgaggccgag tgcgaaatgg gcgtgatggg accaaacggc     480

gaagagatta caccgtctgc gcctcgccaa aatccatggc cgatgacctc catgccttac     540

ggatatggag accggacttt cacggagatc gtcagcaagc tcggtttctc aaacactcct     600

gttccgcagg cacggaacag ccgtccttat gatggccgac cacaatgctg tggtaacaac     660

aactgcatgc caatctgccc gattggcgcg atgtacaacg gcgtatacgc ggcaataaaa     720

gcggaaaagc tgggcgcaaa aattatcccc aatgctgtcg tctacgcgat ggaaacggat     780

gcaaaaaacc gcattacagc gattagcttc tatgatcccg acaagcagtc ccatcgcgtc 840

gttgcaaaaa cattcgtgat cgccgcaaac gggatcgaaa caccgaaact gctgcttctg 900

gcggcaaatg atcgaaaccc tcatgggatt gccaactcat cagaccttgt tggccggaac 960

atgatggacc atccgggcat cggcatgagc ttccagtctg cggagcccat ctgggctggt 1020

ggaggctcag tccagatgag ttccatcacc aacttccgtg atggcgattt ccgctcggag 1080

tatgctgcca cgcagatcgg ctacaacaat acggctcaga actcccgcgc cggcatgaaa 1140

gccctgtcca tggggttggt tggcaagaag ctggatgaag aaatccgccg tcgcacggcg 1200

catggtgtgg acatttatgc caaccatgaa gtcctcccgg accccaacaa ccgtcttgtt 1260

ctctccaaag accataagga tgcgctcggt attccacatc ctgaagtcac ctacgatgtt 1320

ggggagtatg ttcgaaagtc agctgtcatc tcacgacagc gcctgatgga tatcgccaaa 1380

gccatgggcg gtacggaaat cgagatgact ccgtatttta cgcccaacaa ccacatcacc 1440

ggtggcacta tcatgggcca tgatccacgg gattcagtgg tcgataaatg gctccggacc 1500

catgatcatt ccaatctgtt ccttgcaaca ggcgccacaa tggcagcgtc cggtacggtc 1560

aattcaacgt taacaatggc cgcactgtca ttacgcgcgg cagatgccat tctcaatgac 1620

ctgaaacagg ggtaa 1635

<210> 6
<211> 544
<212> PRT
<213> Gluconobacter japonicus

<400> 6

Met Ser Asn Glu Thr Leu Ser Ala Asp Val Val Ile Ile Gly Ala Gly
1               5                   10                  15

Ile Cys Gly Ser Leu Leu Ala His Lys Leu Val Arg Asn Gly Leu Ser
            20                  25                  30

Val Leu Leu Leu Asp Ala Gly Pro Arg Arg Asp Arg Ser Gln Ile Val
        35                  40                  45

Glu Asn Trp Arg Asn Met Pro Pro Asp Asn Lys Ser Gln Tyr Asp Tyr
    50                  55                  60

Ala Thr Pro Tyr Pro Ser Val Pro Trp Ala Pro His Thr Asn Tyr Phe
65                  70                  75                  80

Pro Asp Asn Asn Tyr Leu Ile Val Lys Gly Pro Asp Arg Thr Ala Tyr
                85                  90                  95

Lys Gln Gly Ile Ile Lys Gly Val Gly Gly Thr Thr Trp His Trp Ala
            100                 105                 110

Ala Ser Ser Trp Arg Tyr Leu Pro Asn Asp Phe Lys Leu His Ser Thr
        115                 120                 125

Tyr Gly Val Gly Arg Asp Tyr Ala Met Ser Tyr Asp Glu Leu Glu Pro
    130                 135                 140

Tyr Tyr Tyr Glu Ala Glu Cys Glu Met Gly Val Met Gly Pro Asn Gly
145                 150                 155                 160

Glu Glu Ile Thr Pro Ser Ala Pro Arg Gln Asn Pro Trp Pro Met Thr
                165                 170                 175

Ser Met Pro Tyr Gly Tyr Gly Asp Arg Thr Phe Thr Glu Ile Val Ser
            180                 185                 190

Lys Leu Gly Phe Ser Asn Thr Pro Val Pro Gln Ala Arg Asn Ser Arg
        195                 200                 205

Pro Tyr Asp Gly Arg Pro Gln Cys Cys Gly Asn Asn Asn Cys Met Pro
    210                 215                 220

Ile Cys Pro Ile Gly Ala Met Tyr Asn Gly Val Tyr Ala Ala Ile Lys
225                 230                 235                 240

Ala Glu Lys Leu Gly Ala Lys Ile Ile Pro Asn Ala Val Val Tyr Ala
                245                 250                 255

Met Glu Thr Asp Ala Lys Asn Arg Ile Thr Ala Ile Ser Phe Tyr Asp
            260                 265                 270

Pro Asp Lys Gln Ser His Arg Val Val Ala Lys Thr Phe Val Ile Ala
        275                 280                 285

Ala Asn Gly Ile Glu Thr Pro Lys Leu Leu Leu Leu Ala Ala Asn Asp
    290                 295                 300

Arg Asn Pro His Gly Ile Ala Asn Ser Ser Asp Leu Val Gly Arg Asn
305                 310                 315                 320

Met Met Asp His Pro Gly Ile Gly Met Ser Phe Gln Ser Ala Glu Pro
                325                 330                 335

Ile Trp Ala Gly Gly Gly Ser Val Gln Met Ser Ser Ile Thr Asn Phe
            340                 345                 350

Arg Asp Gly Asp Phe Arg Ser Glu Tyr Ala Ala Thr Gln Ile Gly Tyr
        355                 360                 365

Asn Asn Thr Ala Gln Asn Ser Arg Ala Gly Met Lys Ala Leu Ser Met
    370                 375                 380

Gly Leu Val Gly Lys Lys Leu Asp Glu Glu Ile Arg Arg Arg Thr Ala
385                 390                 395                 400

His Gly Val Asp Ile Tyr Ala Asn His Glu Val Leu Pro Asp Pro Asn
                405                 410                 415

Asn Arg Leu Val Leu Ser Lys Asp His Lys Asp Ala Leu Gly Ile Pro
            420                 425                 430

His Pro Glu Val Thr Tyr Asp Val Gly Glu Tyr Val Arg Lys Ser Ala
        435                 440                 445

Val Ile Ser Arg Gln Arg Leu Met Asp Ile Ala Lys Ala Met Gly Gly
    450                 455                 460

Thr Glu Ile Glu Met Thr Pro Tyr Phe Thr Pro Asn Asn His Ile Thr
465                 470                 475                 480

Gly Gly Thr Ile Met Gly His Asp Pro Arg Asp Ser Val Val Asp Lys
                485                 490                 495

Trp Leu Arg Thr His Asp His Ser Asn Leu Phe Leu Ala Thr Gly Ala
            500                 505                 510

Thr Met Ala Ala Ser Gly Thr Val Asn Ser Thr Leu Thr Met Ala Ala
        515                 520                 525

Leu Ser Leu Arg Ala Ala Asp Ala Ile Leu Asn Asp Leu Lys Gln Gly
    530                 535                 540

<210>  7
<211>  310
<212>  DNA
<213>  Gluconobacter oxydans

<400>  7
gatgttcctg gatctgttgt tctgatgcat cacggtgact tccggacaaa gcacatggat      60

gggcgtcaag ggtcaagatg tcagaaggtt catcttgctc acgtcactgt gaggactttc     120

acgtcgtcgt gacttgagga aaggaaggac ttatgattga gcgttcaaga aaataatgcg     180

cttgcaggcc acttgggctt acgataagga caccgcttcc gcgcacgcgt ccgggccacg     240

agtggcatgc ccggccgtgc agagtctctg caggatgcag gaccaatcgt ttacggcgag 300

ggagaccgaa 310

<210> 8
<211> 190
<212> DNA
<213> Gluconobacter oxydans

<400> 8
gttgatgttc ctgtctgaaa cggccccgca caggcgtcgg gcgcgaacgg tctgtgttgc 60

ggcaggatac cattgtcgcg acatggggca atgcagacgt cgtttttgtt gcggtaactg 120

gataccgcat ggctgaaaac ggcggaaaac cgctgcgaat cacataattc aatgtgtgaa 180

cggggcttga 190

<210> 9
<211> 2427
<212> DNA
<213> Gluconobacter oxydans

<400> 9
atgagcacaa catcccggcc agggctctgg gccctgatta cggccgcggt attcgcgctt 60

tgcggcgcga tccttaccgt tggcggcgca tgggtcgctg ccatcggcgg ccctctctat 120

tatgtcatcc ttggcctggc acttctcgcc acggctttcc tctcattcag gcgcaacccg 180

gctgccctct acctgttcgc agtcgtcgtc ttcggaacgg tcatctggga actcaccgtt 240

gtcggtctcg acatctgggc cctgatcccg cgctcggaca tcgtcatcat cctcggcatc 300

tggctgctgt tgccgttcgt ctcccgccag atcggcggca cgcggacgac cgtcctgccg 360

ctcgccggtg ccgttggcgt tgcggttctg gcgctgttcg ccagcctctt caccgacccg 420

catgacatca gtggcgacct gccgacgcag atcgcaaacg cctcccccgc cgacccggac 480

aacgtcccgg ccagcgagtg gcacgcctat ggtcgtacgc aggccggtga ccgctggtcc 540

ccgctgaacc agatcaacgc gtccaacgtc agcaacctca aggtcgcatg gcatatccac 600

accaaggata tgatgaactc caacgacccg ggcgaagcga cgaacgaagc gaccccgatc 660

gagttcaaca acacgcttta tatgtgctcg ctgcaccaga agctgtttgc ggttgatggt 720

gccaccggta acgtcaagtg ggtctacgat ccgaagctcc agatcaaccc tggcttccag 780

catctgacct gccgtggcgt cagcttccac gaaacgccgg ccaatgccac ggattccgat 840

ggcaatcctg ctccgacgga ctgcgccaag cgcatcatcc tgccggtcaa tgatggccgt 900

ctggttgaag tcgatgccga cacgggcaag acctgctccg gcttcggcaa caatggcgag 960

atcgatctgc gcgttccgaa ccagccttac acgacgcccg gccagtacga gccgacgtcc 1020

ccgccggtca tcacggacaa gctgatcatc gccaacagcg ccatcaccga taacggttcg 1080

gtcaagcagg cttcgggcgc cacgcaggca ttcgacgtct acaccggcaa gcgtgtctgg 1140

gtgttcgatg cgtccaaccc ggacccgaac cagcttccgg atgacagcca ccctgtcttc 1200

cacccgaact ccccgaactc ctggatcgtg tcgtcttacg acaggaacct gaacctcgtg 1260

tacatcccga tgggcgtggg tactcccgat cagtggggcg gtgaccgcac gaaggattcc 1320

gagcgtttcg ctccgggtat cgttgctctg aacgcagaca cgggcaagct cgcctggttc 1380

taccagaccg ttcatcacga tctgtgggac atggacgttc cgtcacagcc gagcctcgtg 1440

gacgtaacgc agaaggacgg cacgcttgtt ccggccatct acgctccgac caagaccggc 1500

gacatcttcg tcctcgaccg tcgtaccggc aaggaaatcg tcccggctcc ggaaaccccg 1560

gttccccagg gcgccgctcc gggcgatcac accagcccga cccagccgat gtcgcagctg 1620

accctgcgtc cgaagaaccc gctgaacgac tccgatatct ggggcggcac gatcttcgac 1680

cagatgttct gcagcatcta tttccacacc ctgcgctacg aaggcccctt cacgccgccg 1740

tcgctcaagg gctcgctcat cttcccgggc gatctgggaa tgttcgagtg gggtggtctg 1800

gccgtcgatc cgcagcgtca ggtggctttc gccaacccga tctccctgcc gttcgtctcc 1860

cagcttgttc cccgtggacc gggcaacccg ctctggcctg aaaaggacgc caagggtacg 1920

ggtggtgaaa ccggcctgca gcacaactat ggcattccgt atgccgtcaa cctgcatccg 1980

ttcctggatc cggtgctgct gccgttcggc atcaagatgc cctgccgtac gccgccctgg 2040

ggctatgtcg ccggtattga cctgaagacc aacaaggtcg tctggcagca ccgcaacggc 2100

accctgcgtg actcgatgta tggcagctcc ctgccgatcc cgctgccgcc gatcaagatc 2160

ggtgtcccga gcctcggtgg cccgctctcc acggctggca acctcggctt cctgacggcg 2220

tccatggatt actacatccg tgcgtacaac ctgacgacgg gcaaggtgct gtggcaggac 2280

cgtctgccgg ctggcgccca ggcaacgccg atcacctatg ccatcaacgg caagcagtac 2340

atcgtgacct atgcaggcgg acacaactcg ttcccgaccc gcatgggcga cgacatcatc 2400

gcctacgccc tgcccgatca gaaatga 2427

<210> 10
<211> 808
<212> PRT
<213> Gluconobacter oxydans

<400> 10

Met Ser Thr Thr Ser Arg Pro Gly Leu Trp Ala Leu Ile Thr Ala Ala
1               5                   10                  15

Val Phe Ala Leu Cys Gly Ala Ile Leu Thr Val Gly Gly Ala Trp Val
            20                  25                  30

Ala Ala Ile Gly Gly Pro Leu Tyr Tyr Val Ile Leu Gly Leu Ala Leu
        35                  40                  45

Leu Ala Thr Ala Phe Leu Ser Phe Arg Arg Asn Pro Ala Ala Leu Tyr
    50                  55                  60

Leu Phe Ala Val Val Val Phe Gly Thr Val Ile Trp Glu Leu Thr Val
65                  70                  75                  80

Val Gly Leu Asp Ile Trp Ala Leu Ile Pro Arg Ser Asp Ile Val Ile
                85                  90                  95

Ile Leu Gly Ile Trp Leu Leu Leu Pro Phe Val Ser Arg Gln Ile Gly
            100                 105                 110

Gly Thr Arg Thr Thr Val Leu Pro Leu Ala Gly Ala Val Gly Val Ala
        115                 120                 125

Val Leu Ala Leu Phe Ala Ser Leu Phe Thr Asp Pro His Asp Ile Ser
    130                 135                 140

Gly Asp Leu Pro Thr Gln Ile Ala Asn Ala Ser Pro Ala Asp Pro Asp
145                 150                 155                 160

Asn Val Pro Ala Ser Glu Trp His Ala Tyr Gly Arg Thr Gln Ala Gly
                165                 170                 175

Asp Arg Trp Ser Pro Leu Asn Gln Ile Asn Ala Ser Asn Val Ser Asn
            180                 185                 190

Leu Lys Val Ala Trp His Ile His Thr Lys Asp Met Met Asn Ser Asn
        195                 200                 205

Asp Pro Gly Glu Ala Thr Asn Glu Ala Thr Pro Ile Glu Phe Asn Asn
    210                 215                 220

Thr Leu Tyr Met Cys Ser Leu His Gln Lys Leu Phe Ala Val Asp Gly
225                 230                 235                 240

Ala Thr Gly Asn Val Lys Trp Val Tyr Asp Pro Lys Leu Gln Ile Asn
                245                 250                 255

Pro Gly Phe Gln His Leu Thr Cys Arg Gly Val Ser Phe His Glu Thr
            260                 265                 270

Pro Ala Asn Ala Thr Asp Ser Asp Gly Asn Pro Ala Pro Thr Asp Cys
        275                 280                 285

Ala Lys Arg Ile Ile Leu Pro Val Asn Asp Gly Arg Leu Val Glu Val

290                 295                 300

Asp Ala Asp Thr Gly Lys Thr Cys Ser Gly Phe Gly Asn Asn Gly Glu
305                 310                 315                 320

Ile Asp Leu Arg Val Pro Asn Gln Pro Tyr Thr Thr Pro Gly Gln Tyr
                325                 330                 335

Glu Pro Thr Ser Pro Pro Val Ile Thr Asp Lys Leu Ile Ile Ala Asn
            340                 345                 350

Ser Ala Ile Thr Asp Asn Gly Ser Val Lys Gln Ala Ser Gly Ala Thr
        355                 360                 365

Gln Ala Phe Asp Val Tyr Thr Gly Lys Arg Val Trp Val Phe Asp Ala
    370                 375                 380

Ser Asn Pro Asp Pro Asn Gln Leu Pro Asp Asp Ser His Pro Val Phe
385                 390                 395                 400

His Pro Asn Ser Pro Asn Ser Trp Ile Val Ser Ser Tyr Asp Arg Asn
                405                 410                 415

Leu Asn Leu Val Tyr Ile Pro Met Gly Val Gly Thr Pro Asp Gln Trp
            420                 425                 430

Gly Gly Asp Arg Thr Lys Asp Ser Glu Arg Phe Ala Pro Gly Ile Val
        435                 440                 445

Ala Leu Asn Ala Asp Thr Gly Lys Leu Ala Trp Phe Tyr Gln Thr Val
    450                 455                 460

His His Asp Leu Trp Asp Met Asp Val Pro Ser Gln Pro Ser Leu Val
465                 470                 475                 480

Asp Val Thr Gln Lys Asp Gly Thr Leu Val Pro Ala Ile Tyr Ala Pro
                485                 490                 495

Thr Lys Thr Gly Asp Ile Phe Val Leu Asp Arg Arg Thr Gly Lys Glu
            500                 505                 510

Ile Val Pro Ala Pro Glu Thr Pro Val Pro Gln Gly Ala Ala Pro Gly
        515                 520                 525

Asp His Thr Ser Pro Thr Gln Pro Met Ser Gln Leu Thr Leu Arg Pro
    530                 535                 540

Lys Asn Pro Leu Asn Asp Ser Asp Ile Trp Gly Gly Thr Ile Phe Asp
545                 550                 555                 560

Gln Met Phe Cys Ser Ile Tyr Phe His Thr Leu Arg Tyr Glu Gly Pro
                565                 570                 575

Phe Thr Pro Pro Ser Leu Lys Gly Ser Leu Ile Phe Pro Gly Asp Leu
            580                 585                 590

Gly Met Phe Glu Trp Gly Gly Leu Ala Val Asp Pro Gln Arg Gln Val
        595                 600                 605

Ala Phe Ala Asn Pro Ile Ser Leu Pro Phe Val Ser Gln Leu Val Pro
    610                 615                 620

Arg Gly Pro Gly Asn Pro Leu Trp Pro Glu Lys Asp Ala Lys Gly Thr
625                 630                 635                 640

Gly Gly Glu Thr Gly Leu Gln His Asn Tyr Gly Ile Pro Tyr Ala Val
                645                 650                 655

Asn Leu His Pro Phe Leu Asp Pro Val Leu Leu Pro Phe Gly Ile Lys
            660                 665                 670

Met Pro Cys Arg Thr Pro Pro Trp Gly Tyr Val Ala Gly Ile Asp Leu
        675                 680                 685

Lys Thr Asn Lys Val Val Trp Gln His Arg Asn Gly Thr Leu Arg Asp
    690                 695                 700

Ser Met Tyr Gly Ser Ser Leu Pro Ile Pro Leu Pro Pro Ile Lys Ile
705                 710                 715                 720

Gly Val Pro Ser Leu Gly Gly Pro Leu Ser Thr Ala Gly Asn Leu Gly
                725                 730                 735

Phe Leu Thr Ala Ser Met Asp Tyr Tyr Ile Arg Ala Tyr Asn Leu Thr
            740                 745                 750

Thr Gly Lys Val Leu Trp Gln Asp Arg Leu Pro Ala Gly Ala Gln Ala
        755                 760                 765

Thr Pro Ile Thr Tyr Ala Ile Asn Gly Lys Gln Tyr Ile Val Thr Tyr
    770                 775                 780

Ala Gly Gly His Asn Ser Phe Pro Thr Arg Met Gly Asp Asp Ile Ile
785                 790                 795                 800

Ala Tyr Ala Leu Pro Asp Gln Lys
                805

<210>  11
<211>  801
<212>  DNA
<213>  Gluconobacter oxydans

<400>  11
atgcctgccc cttacaaaga ccgtttcgcc ggcaagaaag tcctcgtcac cggggcctcc      60

cagggaattg gcgaggccac cgcgcttcgt tttgccgaag aaggcgcgca ggtcgccctc     120

aacggccgca aggaagacaa gctgatcgcc gtccgcgaga agctgcccaa ggtttccggc     180

ggagagcacc cgatcgccac gggtgacatt tccaaagaag acgacgtcaa acgtctggtt     240

gccgagagca tcaaggccat gggtggtctc gacgttctgg tctgcaatgc gggctatcag     300

atcccctccc cgtcagaaga catcaagctc gaagattttg aaggcgtgat ggccgtcaac     360

gtcacggggg tgatgctgcc ctgtcgcgaa gtcatccgct actggctgga aaacggcatc     420

aagggcacga tcattgtgaa ctcctccgtt caccagatca tcccgaaacc gcattatctg     480

ggctattccg cctccaaggg tgccgtcggc aacattgtcc gcacgctggc actggaatat     540

gccacccgcg gcatccgggt gaatgccgtg gcgcccggcg ccatcgtgac gccgatcaac     600

atgtcgtgga tcgacgatcc cgaacagtac aaggccgttt cgagccacat cccgatgaag     660

cgcccgggcg aaagccgcga aatcgcggat gccatcacct tcctcgccgc cgaggacagc     720

acctacatca cgggtcagac cctgtatgtc gatggtggtc tgacgctcta cggcgatttc     780

gaaaacaact ggtcctcgta a                                               801

<210>  12
<211>  266
<212>  PRT
<213>  Gluconobacter oxydans

<400>  12

Met Pro Ala Pro Tyr Lys Asp Arg Phe Ala Gly Lys Lys Val Leu Val
1               5                   10                  15

Thr Gly Ala Ser Gln Gly Ile Gly Glu Ala Thr Ala Leu Arg Phe Ala
            20                  25                  30

Glu Glu Gly Ala Gln Val Ala Leu Asn Gly Arg Lys Glu Asp Lys Leu
        35                  40                  45

Ile Ala Val Arg Glu Lys Leu Pro Lys Val Ser Gly Gly Glu His Pro
    50                  55                  60

Ile Ala Thr Gly Asp Ile Ser Lys Glu Asp Asp Val Lys Arg Leu Val
65                  70                  75                  80

Ala Glu Ser Ile Lys Ala Met Gly Gly Leu Asp Val Leu Val Cys Asn
                85                  90                  95

Ala Gly Tyr Gln Ile Pro Ser Pro Ser Glu Asp Ile Lys Leu Glu Asp
            100                 105                 110

Phe Glu Gly Val Met Ala Val Asn Val Thr Gly Val Met Leu Pro Cys
        115                 120                 125

Arg Glu Val Ile Arg Tyr Trp Leu Glu Asn Gly Ile Lys Gly Thr Ile
    130                 135                 140

Ile Val Asn Ser Ser Val His Gln Ile Ile Pro Lys Pro His Tyr Leu
145                 150                 155                 160

Gly Tyr Ser Ala Ser Lys Gly Ala Val Gly Asn Ile Val Arg Thr Leu
                165                 170                 175

Ala Leu Glu Tyr Ala Thr Arg Gly Ile Arg Val Asn Ala Val Ala Pro
            180                 185                 190

Gly Ala Ile Val Thr Pro Ile Asn Met Ser Trp Ile Asp Asp Pro Glu
        195                 200                 205

Gln Tyr Lys Ala Val Ser Ser His Ile Pro Met Lys Arg Pro Gly Glu
    210                 215                 220

Ser Arg Glu Ile Ala Asp Ala Ile Thr Phe Leu Ala Ala Glu Asp Ser
225                 230                 235                 240

Thr Tyr Ile Thr Gly Gln Thr Leu Tyr Val Asp Gly Gly Leu Thr Leu
                245                 250                 255

Tyr Gly Asp Phe Glu Asn Asn Trp Ser Ser
            260                 265

<210>  13
<211>  1242
<212>  DNA
<213>  Zymomonas mobilis

<400>  13
atgtttaatt ttaatgccag tcgctggacg cgagcccaag cgatgaaagt gaataaattt      60

gatttgacga cctctatgcc ggaaatcggc actgattttc ctattatgcg tgatgacttg      120

tggctgtggg atacttggcc attacgggat atcaatggca atcctgtcag ctttaaaggc      180

tggaatgtta ttttctcttt ggtcgctgac cgcaatattc cgtggaatga tcgccattct 240

catgcccgca tcggctattt ctattctaaa gatggtaaaa gctgggttta tggtggccat 300

ctattgcaag aattggccaa tagccgcacg gcagaatggt ccggcggcac gattatggca 360

ccgggttccc gtaatcaggt cgaaaccttc tttacctcga ctttattcga caagaatggc 420

gtcagagaag ccgttgctgc tgttacgaaa ggccgcattt atgcggatag tgaaggcgtt 480

tggttcaagg gttttgacca gtcaacggat ttgtttcagg ctgatggtct gttttatcaa 540

aattatgcag aaaataatct ctggaatttc cgtgatcctc atgttttcat caatcctgag 600

gatggtgaga cctatgcttt gttcgaagcc aatgttgcca ctgtccgtgg agaagacgat 660

ataggcgagg atgaaattgg ccctgttccg gccaatacgg tcgttccaaa agatgccaat 720

ttatgttcgg cctctattgg tattgcgcgt tgtttgtcgc cggatcgcac cgaatgggag 780

ctgttaccgc ctttgttgac agcctttggc gtcaatgacc agatggaacg gcctcatgtc 840

attttccaga atggtttgac ctatctcttt acgatcagcc atgattcgac ttatgccgat 900

ggcttaaccg gttccgatgg tctttatggc tttgtttccg aaaatggtat ttttggcccc 960

tatgagccgc tgaatggttc tggccttgtt ctcggtggcc ctgcgtcaca gccgaccgaa 1020

gcctatgctc attacatcat gaataatggc ttggttgaat cttttatcaa tgaaatcatt 1080

gatcccaaaa gcggcaaagt cattgcgggc ggtagcttgg caccgacggt tcgcgttgaa 1140

ttacagggac atgagacttt cgcgaccgaa gtctttgatt atggatatat tcccgcatct 1200

tatgcttggc cggtatggcc tttcccggat cgtcgcaaat aa 1242

<210> 14
<211> 413
<212> PRT
<213> Zymomonas mobilis

<400> 14

Met Phe Asn Phe Asn Ala Ser Arg Trp Thr Arg Ala Gln Ala Met Lys
1               5                   10                  15

Val Asn Lys Phe Asp Leu Thr Thr Ser Met Pro Glu Ile Gly Thr Asp
            20                  25                  30

Phe Pro Ile Met Arg Asp Asp Leu Trp Leu Trp Asp Thr Trp Pro Leu
        35                  40                  45

Arg Asp Ile Asn Gly Asn Pro Val Ser Phe Lys Gly Trp Asn Val Ile
    50                  55                  60

Phe Ser Leu Val Ala Asp Arg Asn Ile Pro Trp Asn Asp Arg His Ser
65                  70                  75                  80

His Ala Arg Ile Gly Tyr Phe Tyr Ser Lys Asp Gly Lys Ser Trp Val
                85                  90                  95

Tyr Gly Gly His Leu Leu Gln Glu Leu Ala Asn Ser Arg Thr Ala Glu
            100                 105                 110

Trp Ser Gly Gly Thr Ile Met Ala Pro Gly Ser Arg Asn Gln Val Glu
        115                 120                 125

Thr Phe Phe Thr Ser Thr Leu Phe Asp Lys Asn Gly Val Arg Glu Ala
    130                 135                 140

Val Ala Ala Val Thr Lys Gly Arg Ile Tyr Ala Asp Ser Glu Gly Val
145                 150                 155                 160

Trp Phe Lys Gly Phe Asp Gln Ser Thr Asp Leu Phe Gln Ala Asp Gly
                165                 170                 175

Leu Phe Tyr Gln Asn Tyr Ala Glu Asn Asn Leu Trp Asn Phe Arg Asp
            180                 185                 190

Pro His Val Phe Ile Asn Pro Glu Asp Gly Glu Thr Tyr Ala Leu Phe
        195                 200                 205

Glu Ala Asn Val Ala Thr Val Arg Gly Glu Asp Asp Ile Gly Glu Asp
    210                 215                 220

Glu Ile Gly Pro Val Pro Ala Asn Thr Val Val Pro Lys Asp Ala Asn
225                 230                 235                 240

Leu Cys Ser Ala Ser Ile Gly Ile Ala Arg Cys Leu Ser Pro Asp Arg
                245                 250                 255

Thr Glu Trp Glu Leu Leu Pro Pro Leu Leu Thr Ala Phe Gly Val Asn
            260                 265                 270

Asp Gln Met Glu Arg Pro His Val Ile Phe Gln Asn Gly Leu Thr Tyr
        275                 280                 285

Leu Phe Thr Ile Ser His Asp Ser Thr Tyr Ala Asp Gly Leu Thr Gly
    290                 295                 300

Ser Asp Gly Leu Tyr Gly Phe Val Ser Glu Asn Gly Ile Phe Gly Pro
305                 310                 315                 320

Tyr Glu Pro Leu Asn Gly Ser Gly Leu Val Leu Gly Gly Pro Ala Ser

325                 330                 335

Gln Pro Thr Glu Ala Tyr Ala His Tyr Ile Met Asn Asn Gly Leu Val
            340                 345                 350

Glu Ser Phe Ile Asn Glu Ile Ile Asp Pro Lys Ser Gly Lys Val Ile
        355                 360                 365

Ala Gly Gly Ser Leu Ala Pro Thr Val Arg Val Glu Leu Gln Gly His
    370                 375                 380

Glu Thr Phe Ala Thr Glu Val Phe Asp Tyr Gly Tyr Ile Pro Ala Ser
385                 390                 395                 400

Tyr Ala Trp Pro Val Trp Pro Phe Pro Asp Arg Arg Lys
                405                 410

<210>  15
<211>  1362
<212>  DNA
<213>  Gluconobacter oxydans

<400>  15
atgtccttca ttcccaatac cgaagccgag gccctgagcg cgctgttttc gcgccgctcc      60

gttctgggag cgaccgccgc gggcggtctc ctggcgacgc ctctggcggc ctttgctcag     120

tccggtgcgg cctccggccc cggtgaagca gaaatcaccg tcgatcaggc ccgtcaggag     180

ccgattccca tcgttgttcc gaatttcggg gcagggctgg gtgagcagat ctccggcgtg     240

atcacgtccg acctgaacgg aaccggtctg ttcaaggtca tgagcggcag tgtgccgccc     300

ggctctacgc cggatttcag cgcgctcaaa gcccagggcg cccgcgcggc cgtggccggt     360

caggccgttg gtgcgggaag tgtgcgcgtc gagatgcgtc tgtgggatgt gctctccgga     420

cagcagcttc agggcacggc ctatacggcg tccaacagca actggcgccg gattgcgcat     480

atcattgctg acgtgatcta tgagcggatg ctgggtgaga agggctattt cgatacccgc     540

atcgcctata tcgcccgcac cggtccgcgc catcaccaga tcacgcgtct ggccctgatg     600

gatcaggatg gcgccaacga gcgcatgctg acgggtggcg agtggctgac gctgacgccg     660

cgtttcaacc cggtgcgcga tcagatcgcg ttcatgtcct acgccaacaa ccgtccgcgc     720

gtgtatctgt tcgatctggc ttctggccgg cagcagattc tgggcgagtt cgcgggcatt     780

tcctttgcgc cgcgcttctc gccgacaggt ggctcggtgg tgctgtccgc tacgcgcggg     840

ggcgggtctg acatcttcgt tgtcgatctg gcttcccgtg cgaagcgaca gatcaccaat     900

tccaatggcg cgatcgacac cagcccgtgc ttcagcccgg atggtagcca gatcgtcttc     960

aactccgatc gtggtggcag cccccagctc tacatcatga gtgcatccgg tggggcggcg    1020

aagcggattt cctatggcag tggccagtat ggatccccgg tctggtcccc gcgtggagat 1080

ctgatcgcct ttacccggat tggcagcggc gggttctcgc tcggtgtcat gaacccggat 1140

ggtacgggcg agcgtatcct gacgcagggc ttcacggttg atggggcgac gttctgtccg 1200

aacggtcgtg tgctggcgtt ctgccgccag agcgcgtccg gtgcgggcgg tgcgggtttt 1260

gcgtccggga ttggtacgat cgatattacg ggcttcaacg agcgtccgat ccggatctcg 1320

acgggggcgt ccgacccggc ctggtctccg cggaacggct ga 1362

<210> 16
<211> 453
<212> PRT
<213> Gluconobacter oxydans

<400> 16

Met Ser Phe Ile Pro Asn Thr Glu Ala Glu Ala Leu Ser Ala Leu Phe
1               5                   10                  15

Ser Arg Arg Ser Val Leu Gly Ala Thr Ala Ala Gly Gly Leu Leu Ala
            20                  25                  30

Thr Pro Leu Ala Ala Phe Ala Gln Ser Gly Ala Ala Ser Gly Pro Gly
        35                  40                  45

Glu Ala Glu Ile Thr Val Asp Gln Ala Arg Gln Glu Pro Ile Pro Ile
    50                  55                  60

Val Val Pro Asn Phe Gly Ala Gly Leu Gly Glu Gln Ile Ser Gly Val
65                  70                  75                  80

Ile Thr Ser Asp Leu Asn Gly Thr Gly Leu Phe Lys Val Met Ser Gly
                85                  90                  95

Ser Val Pro Pro Gly Ser Thr Pro Asp Phe Ser Ala Leu Lys Ala Gln
            100                 105                 110

Gly Ala Arg Ala Ala Val Ala Gly Gln Ala Val Gly Ala Gly Ser Val
        115                 120                 125

Arg Val Glu Met Arg Leu Trp Asp Val Leu Ser Gly Gln Gln Leu Gln
    130                 135                 140

Gly Thr Ala Tyr Thr Ala Ser Asn Ser Asn Trp Arg Arg Ile Ala His
145                 150                 155                 160

Ile Ile Ala Asp Val Ile Tyr Glu Arg Met Leu Gly Glu Lys Gly Tyr
                165                 170                 175

Phe Asp Thr Arg Ile Ala Tyr Ile Ala Arg Thr Gly Pro Arg His His
            180                 185                 190

Gln Ile Thr Arg Leu Ala Leu Met Asp Gln Asp Gly Ala Asn Glu Arg
        195                 200                 205

Met Leu Thr Gly Gly Glu Trp Leu Thr Leu Thr Pro Arg Phe Asn Pro
    210                 215                 220

Val Arg Asp Gln Ile Ala Phe Met Ser Tyr Ala Asn Asn Arg Pro Arg
225                 230                 235                 240

Val Tyr Leu Phe Asp Leu Ala Ser Gly Arg Gln Gln Ile Leu Gly Glu
                245                 250                 255

Phe Ala Gly Ile Ser Phe Ala Pro Arg Phe Ser Pro Thr Gly Gly Ser
            260                 265                 270

Val Val Leu Ser Ala Thr Arg Gly Gly Gly Ser Asp Ile Phe Val Val
        275                 280                 285

Asp Leu Ala Ser Arg Ala Lys Arg Gln Ile Thr Asn Ser Asn Gly Ala
    290                 295                 300

Ile Asp Thr Ser Pro Cys Phe Ser Pro Asp Gly Ser Gln Ile Val Phe
305                 310                 315                 320

Asn Ser Asp Arg Gly Gly Ser Pro Gln Leu Tyr Ile Met Ser Ala Ser
                325                 330                 335

Gly Gly Ala Ala Lys Arg Ile Ser Tyr Gly Ser Gly Gln Tyr Gly Ser
            340                 345                 350

Pro Val Trp Ser Pro Arg Gly Asp Leu Ile Ala Phe Thr Arg Ile Gly
        355                 360                 365

Ser Gly Gly Phe Ser Leu Gly Val Met Asn Pro Asp Gly Thr Gly Glu
    370                 375                 380

Arg Ile Leu Thr Gln Gly Phe Thr Val Asp Gly Ala Thr Phe Cys Pro
385                 390                 395                 400

Asn Gly Arg Val Leu Ala Phe Cys Arg Gln Ser Ala Ser Gly Ala Gly
                405                 410                 415

Gly Ala Gly Phe Ala Ser Gly Ile Gly Thr Ile Asp Ile Thr Gly Phe
            420                 425                 430

Asn Glu Arg Pro Ile Arg Ile Ser Thr Gly Ala Ser Asp Pro Ala Trp
        435                 440                 445

Ser Pro Arg Asn Gly
    450

<210>  17
<211>  9326
<212>  DNA
<213>  artificial

<220>
<223>  plasmid pBBR1p264-fdh-strep

<400>  17
taatgaatcg gccaacgcgc ggggagaggc ggtttgcgta ttgggcgcat gcataaaaac      60

tgttgtaatt cattaagcat tctgccgaca tggaagccat cacaaacggc atgatgaacc     120

tgaatcgcca gcggcatcag caccttgtcg ccttgcgtat aatatttgcc catgggggtg      180

ggcgaagaac tccagcatga gatccccgcg ctggaggatc atccagccgg cgtcccggaa     240

aacgattccg aagcccaacc tttcatagaa ggcggcggtg gaatcgaaat ctcgtgatgg     300

caggttgggc gtcgcttggt cggtcatttc gaaccccaga gtcccgctca gaagaactcg     360

tcaagaaggc gatagaaggc gatgcgctgc gaatcgggag cggcgatacc gtaaagcacg     420

aggaagcggt cagcccattc gccgccaagc tcttcagcaa tatcacgggt agccaacgct     480

atgtcctgat agcggtccgc cacacccagc cggccacagt cgatgaatcc agaaaagcgg     540

ccattttcca ccatgatatt cggcaagcag gcatcgccat gggtcacgac gagatcctcg     600

ccgtcgggca tgcgcgcctt gagcctggcg aacagttcgg ctggcgcgag cccctgatgc     660

tcttcgtcca gatcatcctg atcgacaaga ccggcttcca tccgagtacg tgctcgctcg     720

atgcgatgtt tcgcttggtg gtcgaatggg caggtagccg gatcaagcgt atgcagccgc     780

cgcattgcat cagccatgat ggatactttc tcggcaggag caaggtgaga tgacaggaga     840

tcctgccccg gcacttcgcc caatagcagc cagtcccttc ccgcttcagt gacaacgtcg     900

agcacagctg cgcaaggaac gcccgtcgtg gccagccacg atagccgcgc tgcctcgtcc     960

tgcagttcat tcagggcacc ggacaggtcg gtcttgacaa aaagaaccgg gcgcccctgc    1020

gctgacagcc ggaacacggc ggcatcagag cagccgattg tctgttgtgc ccagtcatag    1080

ccgaatagcc tctccaccca agcggccgga gaacctgcgt gcaatccatc ttgttcaatc    1140

atgcgaaacg atcctcatcc tgtctcttga tcagatcttg atcccctgcg ccatcagatc    1200

cttggcggca agaaagccat ccagtttact ttgcagggct tcccaacctt accagagggc    1260

gccccagctg gcaattccgg ttcgcttgct gtccataaaa ccgcccagtc tagctatcgc    1320

catgtaagcc cactgcaagc tacctgcttt ctctttgcgc ttgcgttttc ccttgtccag 1380

atagcccagt agctgacatt catcccaggt ggcacttttc ggggaaatgt gcgcgcccgc 1440

gttcctgctg gcgctgggcc tgtttctggc gctggacttc ccgctgttcc gtcagcagct 1500

tttcgcccac ggccttgatg atcgcggcgg ccttggcctg catatcccga ttcaacggcc 1560

ccagggcgtc cagaacgggc ttcaggcgct cccgaaggtc tcgggccgtc tcttgggctt 1620

gatcggcctt cttgcgcatc tcacgcgctc ctgcggcggc ctgtagggca ggctcatacc 1680

cctgccgaac cgcttttgtc agccggtcgg ccacggcttc cggcgtctca acgcgctttg 1740

agattcccag cttttcggcc aatccctgcg gtgcataggc gcgtggctcg accgcttgcg 1800

ggctgatggt gacgtggccc actggtggcc gctccagggc ctcgtagaac gcctgaatgc 1860

gcgtgtgacg tgccttgctg ccctcgatgc cccgttgcag ccctagatcg gccacagcgg 1920

ccgcaaacgt ggtctggtcg cgggtcatct gcgctttgtt gccgatgaac tccttggccg 1980

acagcctgcc gtcctgcgtc agcggcacca cgaacgcggt catgtgcggg ctggtttcgt 2040

cacggtggat gctggccgtc acgatgcgat ccgccccgta cttgtccgcc agccacttgt 2100

gcgccttctc gaagaacgcc gcctgctgtt cttggctggc cgacttccac cattccgggc 2160

tggccgtcat gacgtactcg accgccaaca cagcgtcctt gcgccgcttc tctggcagca 2220

actcgcgcag tcggcccatc gcttcatcgg tgctgctggc cgcccagtgc tcgttctctg 2280

gcgtcctgct ggcgtcagcg ttgggcgtct cgcgctcgcg gtaggcgtgc ttgagactgg 2340

ccgccacgtt gcccattttc gccagcttct tgcatcgcat gatcgcgtat gccgccatgc 2400

ctgcccctcc cttttggtgt ccaaccggct cgacgggggc agcgcaaggc ggtgcctccg 2460

gcgggccact caatgcttga gtatactcac tagactttgc ttcgcaaagt cgtgaccgcc 2520

tacggcggct gcggcgccct acgggcttgc tctccgggct tcgccctgcg cggtcgctgc 2580

gctcccttgc cagcccgtgg atatgtggac gatggccgcg agcggccacc ggctggctcg 2640

cttcgctcgg cccgtggaca accctgctgg acaagctgat ggacaggctg cgcctgccca 2700

cgagcttgac cacagggatt gcccaccggc tacccagcct tcgaccacat acccaccggc 2760

tccaactgcg cggcctgcgg ccttgcccca tcaatttttt taattttctc tggggaaaag 2820

cctccggcct gcggcctgcg cgcttcgctt gccggttgga caccaagtgg aaggcgggtc 2880

aaggctcgcg cagcgaccgc gcagcggctt ggccttgacg cgcctggaac gacccaagcc 2940

tatgcgagtg ggggcagtcg aaggcgaagc ccgcccgcct gccccccgag cctcacggcg 3000

gcgagtgcgg gggttccaag ggggcagcgc caccttgggc aaggccgaag gccgcgcagt 3060

cgatcaacaa gccccggagg ggccactttt tgccggaggg ggagccgcgc cgaaggcgtg 3120

ggggaacccc gcaggggtgc ccttctttgg gcaccaaaga actagatata gggcgaaatg 3180

cgaaagactt aaaaatcaac aacttaaaaa aggggggtac gcaacagctc attgcggcac 3240

cccccgcaat agctcattgc gtaggttaaa gaaaatctgt aattgactgc cacttttacg 3300

caacgcataa ttgttgtcgc gctgccgaaa agttgcagct gattgcgcat ggtgccgcaa 3360

ccgtgcggca ccctaccgca tggagataag catggccacg cagtccagag aaatcggcat 3420

tcaagccaag aacaagcccg gtcactgggt gcaaacggaa cgcaaagcgc atgaggcgtg 3480

ggccgggctt attgcgagga aacccacggc ggcaatgctg ctgcatcacc tcgtggcgca 3540

gatgggccac cagaacgccg tggtggtcag ccagaagaca ctttccaagc tcatcggacg 3600

ttctttgcgg acggtccaat acgcagtcaa ggacttggtg gccgagcgct ggatctccgt 3660

cgtgaagctc aacggccccg gcaccgtgtc ggcctacgtg gtcaatgacc gcgtggcgtg 3720

gggccagccc cgcgaccagt tgcgcctgtc ggtgttcagt gccgccgtgg tggttgatca 3780

cgacgaccag gacgaatcgc tgttggggca tggcgacctg cgccgcatcc cgaccctgta 3840

tccgggcgag cagcaactac cgaccggccc cggcgaggag ccgcccagcc agcccggcat 3900

tccgggcatg gaaccagacc tgccagcctt gaccgaaacg gaggaatggg aacggcgcgg 3960

gcagcagcgc ctgccgatgc ccgatgagcc gtgttttctg gacgatggcg agccgttgga 4020

gccgccgaca cgggtcacgc tgccgcgccg gtagcacttg ggttgcgcag caacccgtaa 4080

gtgcgctgtt ccagactatc ggctgtagcc gcctcgccgc cctatacctt gtctgcctcc 4140

ccgcgttgcg tcgcggtgca tggagccggg ccacctcgac ctgaatggaa gccggcggca 4200

cctcgctaac ggattcaccg tttttatcag gctctgggag gcagaataaa tgatcatatc 4260

gtcaattatt acctccacgg ggagagcctg agcaaactgg cctcaggcat ttgagaagca 4320

cacggtcaca ctgcttccgg tagtcaataa accggtaaac cagcaataga cataagcggc 4380

tatttaacga ccctgccctg aaccgacgac cgggtcgaat ttgctttcga atttctgcca 4440

ttcatccgct tattatcact tattcaggcg tagcaccagg cgtttaaggg caccaataac 4500

tgccttaaaa aaattacgcc ccgccctgcc actcatcgca gtcggcctat tggttaaaaa 4560

atgagctgat ttaacaaaaa tttaacgcga attttaacaa aatattaacg cttacaattt 4620

ccattcgcca ttcaggctgc gcaactgttg ggaagggcga tcggtgcggg cctcttcgct 4680

attacgccag ctggcgaaag ggggatgtgc tgcaaggcga ttaagttggg taacgccagg 4740

gttttcccag tcacgacgtt gtaaaacgac ggccagtgag cgcgcgtaat acgactcact 4800

atagggcgaa ttggagctcg ttgcgcctga atgagaggaa agccgtggcg agaagtgcca 4860

ggccaaggat gacataatag agaggccgcc cgatggcagc gacccatgcg ccgccaacgg 4920

taaggatcgc gccgcaaagc gcgaataccg cggccgtaat cagggcccag agccctggcc 4980

gggatgttgt gctcatgatg ttcctggatc tgttgttctg atgcatcacg gtgacttccg 5040

gacaaagcac atggatgggc gtcaagggtc aagatgtcag aaggttcatc ttgctcacgt 5100

cactgtgagg actttcacgt cgtcgtgact tgaggaaagg aaggacttat gattgagcgt 5160

tcaagaaaat aatgcgcttg caggccactt gggcttacga taaggacacc gcttccgcgc 5220

acgcgtccgg gccacgagtg gcatgcccgg ccgtgcagag tctctgcagg atgcaggacc 5280

aatcgtttac ggcgagggag accgaatttg ggctgcagga attcgatatc aagcttatcg 5340

attcaggaga aggccaatgg aaaaaatagc tgattccggc cctgttcaaa tctttctttc 5400

gcgtagaaag cttctggctt tttccggtgc ctgcctaaca gtcgcagcaa ttggaacacc 5460

atcgaaagga tctactcagg acgttgtggc attaaatcgc gatagcattt cggattttat 5520

gcagctttct gcttttgcca ccggtcacaa aaatctggat ctcaatatcg gatcagcact 5580

tctcttggca tttgaagctc agaagcatga tttttctgct caaataaagg ctctccgcga 5640

acatattact aaaaataatt atcaggatgt tgaagcactg gatgcagcca tgaaagatga 5700

ccccctgcat cccacgctcc tccagattat ccgggcgtgg tattcaggtg tcatcgaaga 5760

cggaacaaac gctaaagttt acgctttcga aaaagcactc atgtatcagc cgtcccgcga 5820

tgtcgtggtc attccgacat atgctcataa cgggcccaat tactgggtct cggaacccgc 5880

atccgtcgat gtcatgccgg cattttaagg accttattcc gtgattatgc gatattttcg 5940

ccctctgtcc gccacagcca tgacaaccgt tctgcttctc gcaggcacga acgtacgggc 6000

gcaaccgaca gaaccaacac ctgcttcagc ccatagcccc tccatcagcc gcggtcatta 6060

tctggcaatt gccgccgatt gtgcggcctg ccataccaat gggcggaacg gtcagttcct 6120

tgctggcggc tatgccattt cttccccaat gggaaatatt tactcaacca atattacgcc 6180

ctcgaagacg cacggtatcg gcaactatac tctggagcag ttttctaaag cacttcggca 6240

tggtatccgc gccgatggcg ctcaactgta tcccgccatg ccttatgacg cttacaatcg 6300

tctgacggat gaagacgtca aatcgctcta cgcttacatc atgactgaag taaaacccgt 6360

agatgcacct tctcccaaga cgcagctccc cttcccgttt tcaatccgtg catcactcgg 6420

tatttggaaa attgcggcaa ggatcgaagg caaaccctat gtctttgatc atacgcacaa 6480

tgacgactgg aaccgtggtc gctatctggt cgatgagcta gcccattgcg gtgaatgtca 6540

cacaccgcgt aactttcttc tggctcccaa tcagtccgct tatcttgctg gtgcggacat 6600

aggatcatgg cgtgcaccaa atattaccaa tgaccctcaa agcggcattg gcagttggtc 6660

ggatcaggat ctcttccaat atctgaagac tggaaaaacc gcacatgctc gtgcagcagg 6720

cccaatggca gaagcaatcg agcacagcct acaatatctt ccggatgccg atatttctgc 6780

cataattaca tatctgcgtt cggttccggc aaaggcagaa agcggtcaaa cagtcgctaa 6840

ttttgagcat gccggccgcc catccggtta cagtgttgct gacgctaact cccgtcgcac 6900

caacagtact ctaacaaaaa caaccgatgg tgctgctctc tacgaggctg tgtgcgccag 6960

ttgccatcaa tctgacggta aagggtccaa agacggttat tatccgtccc tcgtaggaaa 7020

tacgacgacc gggcagctca atccaaatga tctgatcgcc agtattcttt acggtgtgga 7080

ccgcacaacg gataaccacg aaatcctgat gccggctttt ggtccagact ctctcgtaca 7140

gcccctgaca gatgagcaga tcgccacaat cgcggactat gttctaagcc actttggcaa 7200

tgctcaggcc acagtctcgg cagatgcggt caaacaggtg cgggcgggcg gaaagcaagt 7260

tcctctcgcc aggctcgcca gtccgggcgt gatgctgctt ctcggagctg gtggcatcct 7320

gggagccatt ctggtggtag gcggcctctg gtggctgatc agccgtcgca aaaagcgttc 7380

cgcttaataa gattcaaagg tcaaaatcat gtctaatgaa acgctttcgg ctgatgtcgt 7440

catcattgga gcaggaatct gcggctccct gctcgcccac aaactcgtcc gcaacggact 7500

ttctgtgctg ctcctggatg caggtccccg ccgggaccgc tcccagatcg tagaaaactg 7560

gcgcaacatg ccgcctgaca acaagtccca gtatgattat gcaacacctt accccagcgt 7620

accttgggcc ccgcatacca actacttccc agacaataac tacctgattg ttaaaggccc 7680

ggaccggacg gcttacaagc aaggaatcat caaaggcgtc gggggtacaa cctggcactg 7740

ggcggcttca agctggcggt acctgcccaa cgatttcaag ctgcattcca catatggcgt 7800

gggccgcgat tacgccatga gttacgacga acttgagccg tactattacg aggccgagtg 7860

cgaaatgggc gtgatgggac caaacggcga agagattaca ccgtctgcgc ctcgccaaaa 7920

tccatggccg atgacctcca tgccttacgg atatggagac cggactttca cggagatcgt 7980

cagcaagctc ggtttctcaa acactcctgt tccgcaggca cggaacagcc gtccttatga 8040

tggccgacca caatgctgtg gtaacaacaa ctgcatgcca atctgcccga ttggcgcgat 8100

gtacaacggc gtatacgcgg caataaaagc ggaaaagctg ggcgcaaaaa ttatccccaa 8160

tgctgtcgtc tacgcgatgg aaacggatgc aaaaaaccgc attacagcga ttagcttcta 8220

tgatcccgac aagcagtccc atcgcgtcgt tgcaaaaaca ttcgtgatcg ccgcaaacgg 8280

gatcgaaaca ccgaaactgc tgcttctggc ggcaaatgat cgaaaccctc atgggattgc 8340

caactcatca gaccttgttg gccggaacat gatggaccat ccgggcatcg gcatgagctt 8400

ccagtctgcg gagcccatct gggctggtgg aggctcagtc cagatgagtt ccatcaccaa 8460

cttccgtgat ggcgatttcc gctcggagta tgctgccacg cagatcggct acaacaatac 8520

ggctcagaac tcccgcgccg gcatgaaagc cctgtccatg gggttggttg gcaagaagct 8580

ggatgaagaa atccgccgtc gcacggcgca tggtgtggac atttatgcca accatgaagt 8640

cctcccggac cccaacaacc gtcttgttct ctccaaagac cataaggatg cgctcggtat 8700

tccacatcct gaagtcacct acgatgttgg ggagtatgtt cgaaagtcag ctgtcatctc 8760

acgacagcgc ctgatggata tcgccaaagc catgggcggt acggaaatcg agatgactcc 8820

gtattttacg cccaacaacc acatcaccgg tggcactatc atgggccatg atccacggga 8880

ttcagtggtc gataaatggc tccggaccca tgatcattcc aatctgttcc ttgcaacagg 8940

cgccacaatg gcagcgtccg gtacggtcaa ttcaacgtta acaatggccg cactgtcatt 9000

acgcgcggca gatgccattc tcaatgacct gaaacagggg tacgtaggcg cgccctggag 9060

ccacccgcag ttcgaaaaat aataagcttg acctgtgaag tgaaaaatgg cgcacattgt 9120

gcgacatttt ttttgtctgc cgtttaccgc tactgcgtca cggatctcca cgcgccctgt 9180

agcggcgcat taagcgcggc gggtgtggtg gttacgcgca gcgtgaccgc tacacttgcc 9240

agcgccctag cgcccgctcc tttcgctttc ttcccttcct ttctcgccac gttcgccggc 9300

tttccccgtc aagctctaaa tcggat 9326

<210> 18
<211> 6986
<212> DNA
<213> artificial

<220>
<223> plasmid pBBR1p264-sacC-strep

<400> 18
ctcgggccgt ctcttgggct tgatcggcct tcttgcgcat ctcacgcgct cctgcggcgg 60

cctgtagggc aggctcatac ccctgccgaa ccgcttttgt cagccggtcg gccacggctt 120

ccggcgtctc aacgcgcttt gagattccca gcttttcggc caatccctgc ggtgcatagg 180

cgcgtggctc gaccgcttgc gggctgatgg tgacgtggcc cactggtggc cgctccaggg 240

cctcgtagaa cgcctgaatg cgcgtgtgac gtgccttgct gccctcgatg ccccgttgca 300

gccctagatc ggccacagcg gccgcaaacg tggtctggtc gcgggtcatc tgcgctttgt 360

tgccgatgaa ctccttggcc gacagcctgc cgtcctgcgt cagcggcacc acgaacgcgg 420

tcatgtgcgg gctggtttcg tcacggtgga tgctggccgt cacgatgcga tccgccccgt 480

acttgtccgc cagccacttg tgcgccttct cgaagaacgc cgcctgctgt tcttggctgg 540

ccgacttcca ccattccggg ctggccgtca tgacgtactc gaccgccaac acagcgtcct 600

tgcgccgctt ctctggcagc aactcgcgca gtcggcccat cgcttcatcg gtgctgctgg 660

ccgcccagtg ctcgttctct ggcgtcctgc tggcgtcagc gttgggcgtc tcgcgctcgc 720

ggtaggcgtg cttgagactg gccgccacgt tgcccatttt cgccagcttc ttgcatcgca 780

tgatcgcgta tgccgccatg cctgcccctc ccttttggtg tccaaccggc tcgacggggg 840

cagcgcaagg cggtgcctcc ggcgggccac tcaatgcttg agtatactca ctagactttg 900

cttcgcaaag tcgtgaccgc ctacggcggc tgcggcgccc tacgggcttg ctctccgggc 960

ttcgccctgc gcggtcgctg cgctcccttg ccagcccgtg gatatgtgga cgatggccgc 1020

gagcggccac cggctggctc gcttcgctcg gcccgtggac aaccctgctg gacaagctga 1080

tggacaggct gcgcctgccc acgagcttga ccacagggat tgcccaccgg ctacccagcc 1140

ttcgaccaca tacccaccgg ctccaactgc gcggcctgcg gccttgcccc atcaattttt 1200

ttaattttct ctggggaaaa gcctccggcc tgcggcctgc gcgcttcgct tgccggttgg 1260

acaccaagtg gaaggcgggt caaggctcgc gcagcgaccg cgcagcggct tggccttgac 1320

gcgcctggaa cgacccaagc ctatgcgagt gggggcagtc gaaggcgaag cccgcccgcc 1380

tgccccccga gcctcacggc ggcgagtgcg ggggttccaa gggggcagcg ccaccttggg 1440

caaggccgaa ggccgcgcag tcgatcaaca agccccggag gggccacttt ttgccggagg 1500

gggagccgcg ccgaaggcgt gggggaaccc cgcaggggtg cccttctttg ggcaccaaag 1560

aactagatat agggcgaaat gcgaaagact taaaaatcaa caacttaaaa aaggggggta 1620

cgcaacagct cattgcggca ccccccgcaa tagctcattg cgtaggttaa agaaaatctg 1680

taattgactg ccacttttac gcaacgcata attgttgtcg cgctgccgaa aagttgcagc 1740

tgattgcgca tggtgccgca accgtgcggc accctaccgc atggagataa gcatggccac 1800

gcagtccaga gaaatcggca ttcaagccaa gaacaagccc ggtcactggg tgcaaacgga 1860

acgcaaagcg catgaggcgt gggccgggct tattgcgagg aaacccacgg cggcaatgct 1920

gctgcatcac ctcgtggcgc agatgggcca ccagaacgcc gtggtggtca gccagaagac 1980

actttccaag ctcatcggac gttctttgcg gacggtccaa tacgcagtca aggacttggt 2040

ggccgagcgc tggatctccg tcgtgaagct caacggcccc ggcaccgtgt cggcctacgt 2100

ggtcaatgac cgcgtggcgt ggggccagcc ccgcgaccag ttgcgcctgt cggtgttcag 2160

tgccgccgtg gtggttgatc acgacgacca ggacgaatcg ctgttggggc atggcgacct 2220

gcgccgcatc ccgaccctgt atccgggcga gcagcaacta ccgaccggcc ccggcgagga 2280

gccgcccagc cagcccggca ttccgggcat ggaaccagac ctgccagcct tgaccgaaac 2340

ggaggaatgg gaacggcgcg ggcagcagcg cctgccgatg cccgatgagc cgtgttttct 2400

ggacgatggc gagccgttgg agccgccgac acgggtcacg ctgccgcgcc ggtagcactt 2460

gggttgcgca gcaacccgta agtgcgctgt tccagactat cggctgtagc cgcctcgccg 2520

ccctatacct tgtctgcctc cccgcgttgc gtcgcggtgc atggagccgg gccacctcga 2580

cctgaatgga agccggcggc acctcgctaa cggattcacc gtttttatca ggctctggga 2640

ggcagaataa atgatcatat cgtcaattat tacctccacg gggagagcct gagcaaactg 2700

gcctcaggca tttgagaagc acacggtcac actgcttccg gtagtcaata aaccggtaaa 2760

ccagcaatag acataagcgg ctatttaacg accctgccct gaaccgacga ccgggtcgaa 2820

tttgctttcg aatttctgcc attcatccgc ttattatcac ttattcaggc gtagcaccag 2880

gcgtttaagg gcaccaataa ctgccttaaa aaaattacgc cccgccctgc cactcatcgc 2940

agtcggccta ttggttaaaa aatgagctga tttaacaaaa atttaacgcg aattttaaca 3000

aaatattaac gcttacaatt tccattcgcc attcaggctg cgcaactgtt gggaagggcg 3060

atcggtgcgg gcctcttcgc tattacgcca gctggcgaaa gggggatgtg ctgcaaggcg 3120

attaagttgg gtaacgccag ggttttccca gtcacgacgt tgtaaaacga cggccagtga 3180

gcgcgcgtaa tacgactcac tatagggcga attggagctc gttgcgctga atgagaggaa 3240

agccgtggcg agaagtgcca ggccaaggat gacataatag agagggccgc cgatggcagc 3300

gacccatgcg ccgccaacgg taaggatcgc gccgcaaagc gcgaataccg cggccgtaat 3360

cagggcccag agccctggcc gggatgttgt gctcatgatg ttcctggatc tgttgttctg 3420

atgcatcacg gtgacttccg gacaaagcac atggatgggc gtcaagggtc aagatgtcag 3480

aaggttcatc ttgctcacgt cactgtgagg actttcacgt cgtcgtgact tgaggaaagg 3540

aaggacttat gattgagcgt tcaagaaaat aatgcgcttg caggccactt gggcttacga 3600

taaggacacc gcttccgcgc acgcgtccgg gccacgagtg gcatgcccgg ccgtgcagag 3660

tctctgcagg atgcaggacc aatcgtttac ggcgagggag accgaatttg ggctgcagga 3720

attcgatatc aagcttatcg ataccgtcga cctcgaggtt taactttaag aaggagatat 3780

acatatgaaa tacctgctgc cgaccgctgc tgctggtctg ctgctcctcg ctgcccagcc 3840

ggcgatggcc tacgtattta attttaatgc cagtcgctgg acgcgagccc aagcgatgaa 3900

agtgaataaa tttgatttga cgacctctat gccggaaatc ggcactgatt ttcctattat 3960

gcgtgatgac ttgtggctgt gggatacttg gccattacgg gatatcaatg gcaatcctgt 4020

cagctttaaa ggctggaatg ttattttctc tttggtcgct gaccgcaata ttccgtggaa 4080

tgatcgccat tctcatgccc gcatcggcta tttctattct aaagatggta aaagctgggt 4140

ttatggtggc catctattgc aagaattggc caatagccgc acggcagaat ggtccggcgg 4200

cacgattatg gcaccgggtt cccgtaatca ggtcgaaacc ttctttacct cgactttatt 4260

cgacaagaat ggcgtcagag aagccgttgc tgctgttacg aaaggccgca tttatgcgga 4320

tagtgaaggc gtttggttca agggttttga ccagtcaacg gatttgtttc aggctgatgg 4380

tctgttttat caaaattatg cagaaaataa tctctggaat ttccgtgatc ctcatgtttt 4440

catcaatcct gaggatggtg agacctatgc tttgttcgaa gccaatgttg ccactgtccg 4500

tggagaagac gatataggcg aggatgaaat tggccctgtt ccggccaata cggtcgttcc 4560

aaaagatgcc aatttatgtt cggcctctat tggtattgcg cgttgtttgt cgccggatcg 4620

caccgaatgg gagctgttac cgcctttgtt gacagccttt ggcgtcaatg accagatgga 4680

acggcctcat gtcattttcc agaatggttt gacctatctc tttacgatca gccatgattc 4740

gacttatgcc gatggcttaa ccggttccga tggtctttat ggctttgttt ccgaaaatgg 4800

tatttttggc ccctatgagc cgctgaatgg ttctggcctt gttctcggtg gccctgcgtc 4860

acagccgacc gaagcctatg ctcattacat catgaataat ggcttggttg aatcttttat 4920

caatgaaatc attgatccca aaagcggcaa agtcattgcg ggcggtagct tggcaccgac 4980

ggttcgcgtt gaattacagg gacatgagac tttcgcgacc gaagtctttg attatggata 5040

tattcccgca tcttatgctt ggccggtatg gcctttcccg gatcgtcgca aaggcgcgcc 5100

cggtggaggt tcagcttgga gccacccgca gttcgaaaaa taataagctt gacctgtgaa 5160

gtgaaaaatg gcgcacattg tgcgacattt tttttgtctg ccgtttaccg ctactgcgtc 5220

acggatctcc acgcgccctg tagcggcgca ttaagcgcgg cgggtgtggt ggttacgcgc 5280

agcgtgaccg ctacacttgc cagcgcccta gcgcccgctc ctttcgcttt cttcccttcc 5340

tttctcgcca cgttcgccgg ctttccccgt caagctctaa atcggattaa tgaatcggcc 5400

aacgcgcggg gagaggcggt ttgcgtattg ggcgcatgca taaaaactgt tgtaattcat 5460

taagcattct gccgacatgg aagccatcac aaacggcatg atgaacctga atcgccagcg 5520

gcatcagcac cttgtcgcct tgcgtataat atttgcccat gggggtgggc gaagaactcc 5580

agcatgagat ccccgcgctg gaggatcatc cagccggcgt cccggaaaac gattccgaag 5640

cccaaccttt catagaaggc ggcggtggaa tcgaaatctc gtgatggcag gttgggcgtc 5700

gcttggtcgg tcatttcgaa ccccagagtc ccgctcagaa gaactcgtca agaaggcgat 5760

agaaggcgat gcgctgcgaa tcgggagcgg cgataccgta aagcacgagg aagcggtcag 5820

cccattcgcc gccaagctct tcagcaatat cacgggtagc caacgctatg tcctgatagc 5880

ggtccgccac acccagccgg ccacagtcga tgaatccaga aaagcggcca ttttccacca 5940

tgatattcgg caagcaggca tcgccatggg tcacgacgag atcctcgccg tcgggcatgc 6000

gcgccttgag cctggcgaac agttcggctg gcgcgagccc ctgatgctct tcgtccagat 6060

catcctgatc gacaagaccg gcttccatcc gagtacgtgc tcgctcgatg cgatgtttcg 6120

cttggtggtc gaatgggcag gtagccggat caagcgtatg cagccgccgc attgcatcag 6180

ccatgatgga tactttctcg gcaggagcaa ggtgagatga caggagatcc tgccccggca 6240

cttcgcccaa tagcagccag tcccttcccg cttcagtgac aacgtcgagc acagctgcgc 6300

aaggaacgcc cgtcgtggcc agccacgata gccgcgctgc ctcgtcctgc agttcattca 6360

gggcaccgga caggtcggtc ttgacaaaaa gaaccgggcg cccctgcgct gacagccgga 6420

acacggcggc atcagagcag ccgattgtct gttgtgccca gtcatagccg aatagcctct 6480

ccacccaagc ggccggagaa cctgcgtgca atccatcttg ttcaatcatg cgaaacgatc 6540

ctcatcctgt ctcttgatca gatcttgatc ccctgcgcca tcagatcctt ggcggcaaga 6600

aagccatcca gtttactttg cagggcttcc caaccttacc agagggcgcc ccagctggca 6660

attccggttc gcttgctgtc cataaaaccg cccagtctag ctatcgccat gtaagcccac 6720

tgcaagctac ctgctttctc tttgcgcttg cgttttccct tgtccagata gcccagtagc 6780

tgacattcat cccaggtggc acttttcggg gaaatgtgcg cgcccgcgtt cctgctggcg 6840

ctgggcctgt ttctggcgct ggacttcccg ctgttccgtc agcagctttt cgcccacggc 6900

cttgatgatc gcggcggcct tggcctgcat atcccgattc aacggcccca gggcgtccag 6960

aacgggcttc aggcgctccc gaaggt 6986